# EUROPEAN PATENT SPECIFICATION

(11) **EP 3 104 829 B1**
(45) Date of publication and mention of the grant of the patent: **22.07.2020**
(21) Application number: 15707170.5
(22) Date of filing: 13.02.2015
(51) Int. Cl.: A61K 8/25, A61K 8/34, A61K 8/36, A61K 8/365, A61K 8/49, A61K 8/60, A61K 8/73, A61Q 15/00, A61K 8/86, A61K 8/04, A61K 8/19

(54) **STABILISED SILICATE COMPOSITIONS AND THEIR USE AS ANTIPERSPIRANT COMPOSITIONS**
SCHWEISSHEMMENDE STABILISIERTE SILICATE ENTHALTENDE ZUSAMMENSETZUNGEN
COMPOSITIONS ANTITRANSPIRANTES COMPRENANT DES SILICATES STABILISÉS

(30) Priority: 14.02.2014 GB 201402669
(43) Date of publication of application: 21.12.2016
(73) Proprietor: United Kingdom Research and Innovation, Swindon SN2 1FL (GB)
(72) Inventor: BASTOS, Carlos André Passos, Cambridge Cambridgeshire CB1 3RY (GB); POWELL, Jonathan, Joseph, Cambridge Cambridgeshire CB4 3JD (GB); FARIA, Nuno, Jorge, Rodrigues, Milton Ernest Bedfordshire MK44 1RX (GB)
(74) Representative: Mewburn Ellis LLP
(86) International application number: PCT/GB2015/050410
(87) International publication number: WO 2015/121667

(56) References cited:
- EP-A1- 0 479 218
- EP-A1- 0 621 038
- EP-A1- 1 132 095
- EP-A1- 2 151 466
- EP-A2- 0 337 464
- US-A1- 2007 148 113

## Description

### Field of the Invention

The present invention relates to devices for delivering an antiperspirant composition comprising a stabilised silicate composition, to a non-therapeutic cosmetic process for treating human body odour using such an antiperspirant composition and to an antiperspirant composition for use in a method of treating of a medical condition characterised by excessive perspiration comprising such a stabilised silicate composition.

### Background of the Invention

Antiperspirants are compounds that inhibit perspiration, typically by blocking sweat from being released out of the body, or in some cases by inhibiting sweat production¹. Antiperspirants are not to be confused with deodorants, which aim to reduce or mask body odour caused by perspiration, rather than reducing perspiration itself. For more than 50 years, aluminium compounds have been the main active ingredient in antiperspirants.

Aluminium-based antiperspirants rely on the chemical properties of this hydrolytic metal, which remains soluble at low pHs but polymerises into a solid mineral when exposed to neutral or mildly acidic conditions present on skin. When used as an antiperspirant, the active aluminium agent is formulated at low pHs, typically pH < 4 (some agents require as low as pH 2), to ensure it remains soluble during storage. Once applied to the skin, the soluble aluminium species diffuse into the eccrine sweat duct where they encounter a pH around 6 which induces hydrolytic polymerisation and subsequently the formation of a gel plug that blocks the pore and that impedes sweat from being released. The key aspect to this mechanical obstruction process is that the aluminium species remain soluble in the antiperspirant formulation (e.g. roll-on) and will only polymerise (and gel) once in the pore.

US 7,303,767 (J.M. Huber Corporation) discloses the use of micro-particulate metal silicates, such as calcium silicate, for use in personal care compositions. These materials consist of large particles (> 1 µm) that are coated with a hydrophilic film for adsorbing smell when used, the coating also serving to protect the users' skin from the high pH level of uncoated calcium silicate. In these compositions, the micro-particulate metal silicates serve as deodorants, while the antiperspirant active ingredients are traditional aluminium or zirconium salts, such as aluminium halides, aluminium hydroxyhalides, zirconyl oxyhalides and zirconyl hydroxy-halides.

US 5,468,473 (Mullen) discloses the use of silicates as gelling agents for antiperspirant sticks. The silicates have a formulation role only and are therefore used in combination with aluminium or zirconium salts, which are effectively the sole antiperspirant actives in those compositions, at a pH in the range of 5.8 to 6.0.

US 2007/148113 (Lemoine et al.) uses colloidal silica (SiO₂) with cation stabilised surfaces, the cations preferably being aluminium, zirconium or hafnium. The material disclosed are fully condensed form of silicic acid, i.e. -OH groups are virtually absent, and thus likely to be biopersistent, an undesirable feature. Furthermore the materials disclosed in US 2007/148113 are stable in their colloid form from pH 4.5 to pH 7 and do not form gels at pH 6.

EP 0 479 218 A relates to highly polymerised silicic acid as a flocculent for water treatment.

EP 0 621 038 A relates to silicate polymers, including polysilicic acids, for pharmaceutical use, generally in the form of tablets, capsules, injection, ointment or suppositories, for use as an anti-allergy agent, an anti-inflammatory agent, an agent for improving peripheral blood circulation, an agent for improving paraesthesia or an analgesic agent.

EP 1 132 095 A relates to compositions that combine a saturated fatty acid and a water-soluble silicate polymer for use as an anti-allergic agent.

EP 2 151 466 A discloses highly condensed silicic acid particles having shell-like structures.

Other hydrolytic metals, with a chemical behaviour similar to that of aluminium, have been attempted as antiperspirants. However, low efficacy (i.e. gel with poor physical properties), high cost, and/or potential toxicity have prevented their development. Currently, zirconium is the only hydrolytic metal, other than aluminium, that is used commercially as an antiperspirant but its use is very limited due to concerns over its toxicity.

The regular use of aluminium-based antiperspirants leads to high levels of this non-essential metal in the skin, which, worryingly, is known to be systemically toxic. Also, since antiperspirants are used regularly, and for decades, the perceived risk associated with their use is steadily rising, and studies have linked antiperspirant usage to breast cancer and Alzheimer's disease^{2,3}. However, there are no current alternatives to aluminium and, thus, the pressure to move to other antiperspirant agents has been so far very limited.

### Summary of the Invention

Broadly, the present invention is based on the inventors' insight that silicates (or polysilicic acid compositions) are highly biocompatible and capable of forming strong, clear gels, which would make them suitable for use in antiperspirants as they would overcome some of the disadvantages associated with the use of aluminium and zirconium salts. However, in order to take advantage of the desirable properties of silicates in forming stable biocompatible gels, the present invention addresses the fundamental problem that the chemistry of silicates does not make them an obvious antiperspirant material as they remain soluble only above pH 10.5 to 11.0 and such caustic solutions cannot be directly applied to the skin. On the other hand, if the pH of the silicate solutions is lowered to more tolerable levels (e.g. pH 8.0), it is found that polymerisation proceeds rapidly and would occur within the formulated antiperspirant material, rather than in sweat pores. Moreover, even if the solubility of silicates could be extended to lower alkalinity, there is the further challenge that the buffering capacity of skin is far lower for moderately alkaline solutions than it is for acidic solutions, such as those of aluminium antiperspirants, so that the pH shift needed to induce polymerisation in sweat pores is harder to achieve than that required when using aluminium and zirconium salts. Accordingly, in one aspect, the present invention concerns an antiperspirant composition comprising a stabilised silicate composition which comprises polysilicic acids, wherein the application of the antiperspirant composition to a subject causes a pH shift that induces growth of the polysilicic acids to form a gel thereby providing an antiperspirant active ingredient.

The present inventors addressed these issues by realising that it is possible to produce silicate compositions in which the silicate is stabilised as polysilicic acid at mildly acidic pHs, such as pH 3.0 to 5.0. The methods disclosed herein mean that it is possible to reduce the kinetics of the growth process of the polysilicic acids that leads to the formation of a gel, so that composition is sufficiently stable to be formulated as an antiperspirant composition. However, when the pH is raised to about pH 6.0 or above, as happens when the composition is applied to the skin, the inhibition of growth is reversed and the silicate composition forms a gel, the reaction typically taking between 5 and 30 minutes. For the avoidance of doubt, the materials disclosed herein form gels *in situ*, i.e. under skin conditions, and are distinct from compositions where silicates are applied as a pre-formed gel. In addition, the antiperspirant compositions of the present invention may have one or more of the following advantages over the prior art, in particular the use of hydrolytic aluminium and zirconium salts.

Firstly, the stabilised silicate compositions of the present invention are different from the colloidal silica (SiO₂) with cation-stabilised surfaces disclosed in US 2007/148113. The latter material is fully condensed and therefore unable to form a gel in response to a change in pH when applied to a subject's skin at pH 6. It is also generally preferred that, in contrast to the colloidal silica (SiO₂) with cation-stabilised surfaces disclosed in US 2007/148113, preferably the polysilicic acids of the present invention have some surface hydroxyl groups present on the surface, even when optionally modified with cations. By way of example, preferably at least 0.01% of the surface oxygen groups will be present as hydroxyl groups (-OH), more preferably at least 0.1%, more preferably at least 1%, more preferably at least 5% and more preferably at least 10%.

The properties of the stabilised silicate compositions of the present invention are well adapted for use as antiperspirants. The safety of the compositions is excellent as polysilicic acids are tolerated by the skin and, unlike aluminium salts, there would be no safety issues regarding systemic toxicity. Moreover, the pH of the initial composition (3.0 to 5.0) and the pH at which gel formation occurs, i.e. pH 6, are at physiologically acceptable conditions. Moreover the gel-forming reaction happens within a short time period, typically between 5 and 30 minutes. The gel produced by the pH shift of the composition is odourless and colourless, and hence does not leave noticeable stains on skin or clothing. Furthermore, amorphous silicate materials are regarded as of low toxicity and any systemically absorbed will be dissolved to silicic acid, which is considered biologically desirable for connective tissue health, for example. The pH shift may be at a pH of above 5 and below 8, preferentially 5.5 to 6.5, and more preferably to a pH of about 6.0.

Accordingly, in one aspect, the present invention provdes a method for producing an antiperspirant composition which comprises stabilised polysilicic acids, the method comprising:
(a) preparing an alkaline silicate solution having a pH ≥ 9.5;
(b) optionally adding a growth retardant to the alkaline silicate solution;
(c) lowering the pH to ≤ 4.0 by adding an acid to form a composition comprising polysilicic acids;
(d) optionally adding a multivalent cation and/or a growth retardant;
(e) raising the pH of the composition to a physiological acceptable pH by adding a base, thereby forming the stabilised composition comprising polysilicic acids;
(f) optionally adding a growth retardant capable of increasing the stability of the composition, wherein the growth retardant is a carboxylic acid, an amino acid, an inorganic anion, a polyol, saccharide or a quaternary ammonium cation; and,
(g) optionally adding a non-aqueous solvent capable of increasing the stability of the composition; and
(h) incorporating the polysilicic acid composition into an antiperspirant formulation;
wherein the polysilicic acid is present in the form of polysilicic acid nanoparticles having mean diameters of 20 nm or less and the antiperspirant composition has a pH between 3.0 and 5.0.

In a further aspect, the present invention provides a device for delivering an antiperspirant composition comprising a stabilised silicate composition which comprises polysilicic acids, wherein the antiperspirant composition is as obtainable by a method comprising providing an alkaline silicate solution having a pH ≥ 9.5 and lowering the pH to between 3.0 and 5.0 prior to application to a subject, wherein the application of the antiperspirant composition to the subject causes a pH shift in the composition that induces growth of the polysilicic acids to form a gel thereby providing an antiperspirant active ingredient, wherein the polysilicic acid is present in the form of polysilicic acid nanoparticles having mean diameters of 20 nm or less, and wherein the device is selected from an aerosol device, a pump-dispenser bottle, a roll-on, a device equipped with a perforated wall, or a wand (stick);
wherein the pH is lowered over a period of less than 60 seconds; and/or
wherein the polysilicic acid composition is stabilised by a growth retardant, wherein the growth retardant is a carboxylic acid, an amino acid, an inorganic anion, a polyol, saccharide or a quaternary ammonium cation; and/or
wherein the polysilicic acid composition is stabilised by a multivalent cation and/or by a non-aqueous solvent.

In a further aspect, the present invention provides a non-therapeutic cosmetic process for treating human body odour using an antiperspirant composition comprising a stabilised silicate composition which comprises polysilicic acids, wherein the antiperspirant composition is as obtainable by a method comprising providing an alkaline silicate solution having a pH ≥ 9.5 and lowering the pH to between 3.0 and 5.0 prior to application to a subject, wherein the application of the antiperspirant composition to the subject causes a pH shift in the composition that induces growth of the polysilicic acids to form a gel thereby providing an antiperspirant active ingredient, wherein the polysilicic acid is present in the form of polysilicic acid nanoparticles having mean diameters of 20 nm or less;
wherein the pH is lowered over a period of less than 60 seconds; and/or
wherein the polysilicic acid composition is stabilised by a growth retardant, wherein the growth retardant is a carboxylic acid, an amino acid, an inorganic anion, a polyol, saccharide or a quaternary ammonium cation; and/or
wherein the polysilicic acid composition is stabilised by a multivalent cation and/or by a non-aqueous solvent.

In a further aspect, the present invention provides an antiperspirant composition for use in a method of treating of a medical condition characterised by excessive perspiration using an antiperspirant composition comprising a stabilised silicate composition which comprises polysilicic acids wherein the antiperspirant composition is as obtainable by a method comprising providing an alkaline silicate solution having a pH ≥ 9.5 and lowering the pH to between 3.0 and 5.0 prior to application to a subject, wherein the application of the antiperspirant composition to the subject causes a pH shift in the composition that induces growth of the polysilicic acids to form a gel thereby providing an antiperspirant active ingredient, wherein the polysilicic acid is present in the form of polysilicic acid nanoparticles having mean diameters of 20 nm or less;
wherein the pH is lowered over a period of less than 60 seconds; and/or
wherein the polysilicic acid composition is stabilised by a growth retardant, wherein the growth retardant is a carboxylic acid, an amino acid, an inorganic anion, a polyol, saccharide or a quaternary ammonium cation; and/or
wherein the polysilicic acid composition is stabilised by a multivalent cation and/or by a non-aqueous solvent.

Embodiments of the present invention will now be described by way of example and not limitation with reference to the accompanying figures. However various further aspects and embodiments of the present invention will be apparent to those skilled in the art in view of the present disclosure.
"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

### Brief Description of the Figures

**Figure 1****.** Solubility of silicic acid in water vs. pH at 25°C.
**Figure 2****.** Effect of pH on silicate solutions. Silicate solutions (0.5 M) remain soluble indefinitely at pH 11.5 but gel almost immediately after being adjusted to pH 8.0. From visual inspection, the gel formed at pH 8.0 was stronger than an aluminium based gel.
**Figure 3****.** Influence of the rate of pH drop on the growth of polysilicic acids. The pH of silicate solutions (0.5 M) was lowered from pH 12 to pH 4 in less than 5 s, 1 min or 2 min. The increase in viscosity is correlated with the gradual growth of polysilicic acids which leads to the formation of a gel.
**Figure 4****.** Growth of polysilicic acids (0.5 M) at pH 4. The solution pH was lowered from pH 12 to pH 4 in less than 5 sec. The increase in viscosity is correlated with the gradual growth of the polysilicic acids and a viscosity above 8 mPa·s corresponds to a fully formed gel.
**Figure 5****.** Increase in viscosity as a function of time in the presence of different retardants. No growth retardant was added to control whereas 0.1 M gluconic acid, 0.1 M adipic acid, or 0.05 M succinic acid were added to a 0.5 M silicate solution prior to lowering to pH 4.
**Figure 6****.** Formation of a gel upon raising pH from 4.0 to 6.0 in a stabilised polysilicic acid composition. The polysilicic acid composition (0.5 M) was stabilized by xylitol (1.5 M) at pH 4 prior to the pH increase. Note that ethanol was not added, as the assay intended to mimic application by a spray, in which the 20% v/v ethanol would have evaporated. Nevertheless, gelling would have occurred at pH 6 regardless of ethanol being present.
**Figure 7****.** Change in particle size upon raising the pH of a non-stabilised suspension of polysilicic acids (0.5 M) from pH 1.0 to pH 4.0.
**Figure 8****.** Transient particle size stability at pH 4.0 of a suspension of polysilicic acids (0.5 M) stabilised with sucrose (1.5 M).
**Figure 9****.** Comparison of the dissolution rates of polysilicic acids (triangles) of the present invention stabilised with PEG (synthesis: 0.5 M Si plus 1.0 M PEG) and commercial condensed silicates (Ludox SM30®). The rate of dissolution was determined by a molybdic acid assay described herein.
**Figure 10****:** *E.coli* growth curves over time in the presence of different stabilised Cu-containing polysilicic acids versus non-stabilised and non-copper containing polysilicic acids.

### Detailed Description

### Stabilised silicate compositions

As is well known in the art, there is an equilibrium between silicic acid and increasingly condensed silicate species, namely di- and tri- silicic acids, polysilicic acids and silica particles. The process of formation and growth from solutions of silicic acid involves growth where the single unit grows in size and in parallel, and depending on synthesis conditions, structural arrangement into more condensed (i.e. less labile, soluble and/or dissolvable) and, thus, less able to return towards Si(OH)₄ in the absence of added alkali. Growth can include polymerisation, agglomeration, aggregation or an increase in size due to surface deposition of soluble species. The growth of polysilicic acids eventually leads to gel formation under suitable conditions. For example, under conditions of pH ≤ 9.5, if Si concentrations are relatively high (∼ ≥ 150 mM), particle species will head towards a gel structure. These factors make it extremely difficult to stabilise silicate compositions above these concentrations of aqueous silicate and at physiologically relevant pHs.

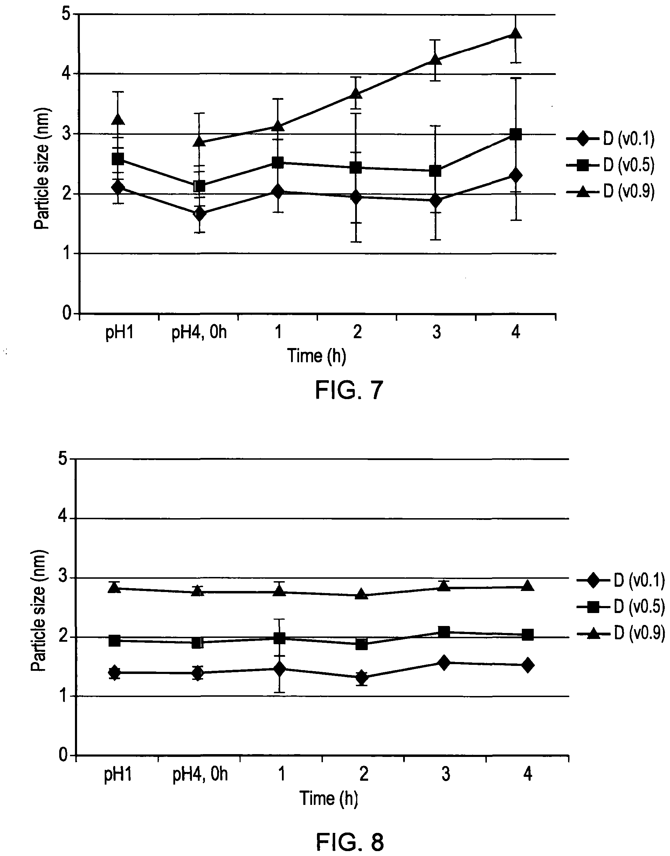

The polysilicic acid compositions used in the present invention comprise silicate in the form of polysilicic acid clusters or polysilicic nanoparticles or colloids. In some cases, the polysilicic acid compositions will be in the form of nanoscaled agglomerates. Polysilicic acid clusters are silicate polymers comprising two or more silicate (silicic acid) monomers. Preferably, the radius of the polymeric clusters is relatively small so that the silicates are relatively uncondensed, for example where the radius of a polymeric cluster is no greater than 10 Å. Preferably, the polysilicic acids used in the present invention are present as a sol, colloidal dispersion or dispersion in which the particulate polysilicic acids are present as discreet, non-aggregated particles that preferably have a mean diameter of 10 nm or less, more preferably 5 nm or less, 4 nm or less, 3 nm or less, 2 nm or less, or 1 nm. The polymeric silicic acid compositions used in the present invention comprise soluble polysilicic acid and nanoparticles of polymeric silicic acid having mean diameters of 20 nm or less, and in some cases mean diameters that are more preferably less than 10 nm, more preferably less than 5 nm, 4 nm, 3 nm, 2 nm or 1 nm. In some embodiments, the particles may range from about 1 nm to about 2 nm, or from about 1 nm to about 3 nm, or from about 1 nm to about 4 nm, or from about 1 nm to about 5 nm, or from about 1 nm to about 10 nm, or from about 1 nm to about 15 nm1 nm, or from about 1 nm to about 20 nm, or from about 5 nm to about 20 nm, or from about 5 nm to about 15 nm, or from about 5 nm to about 10 nm, or from about 10 nm to about 15 nm, or from about 10 nm to about 20 nm, or from about 15 nm to about 20 nm. Generally it is preferred that the particles have mean diameters of 5nm or less. Small sizes of the polymeric polysilicic acids (e.g. less than 5 nm) are generally desirable as they facilitate rapid diffusion of polysilicic acids to the sweat duct increasing antiperspirant efficacy. However, there could be types of use where larger sizes are desirable. These are achievable by a temporary increase in pH of unstabilised polysilicic acids (Figure 7). Long term stability is subsequently achieved by a drop in pH and/or addition of a stabiliser.

The polysilicic acid compositions used in the present invention may be contrasted with more condensed forms of silicates, including larger nanoparticles (e.g. preferably having a mean size greater than 50 nm, and more preferably greater than 20 nm), polysilicic acid gels and silicon dioxide (SiO₂) the fully condensed form of silicic acid, in which -OH groups are virtually absent. The size of the particles of polysilicic acids can be determined using dynamic light scattering and it is preferred that the measurements are made on freshly prepared samples. As will be understood by those skilled in the art, the polysilicic acids will be in equilibrium with other silicate species. For example, and depending on the precise conditions present, this may include small amounts of soluble silicic acid.

Preferably, the polymeric silicates compositions of the present invention have the property of being resorbable, that is that they are poorly condensed amorphous silicates that are capable of undergoing dissolution, within therapeutically useful timescales, upon administration. The amorphous nature of polymeric silicate acid compositions and different levels of condensation and the corresponding structural arrangement of the solid phase that can be exhibited by amorphous mineral phases, may be undistinguishable by XRD analysis (or equivalent). Accordingly, in the present invention, the level of condensation can be determined by appropriate *in vitro* dissolution assays, whereby poorly condensed amorphous silicates exhibit faster dissolution rates as compared to condensed amorphous silicates of equivalent particle size.

In one example, a dissolution assay may involve taking a sample of a polymeric silicate composition and diluting it in buffer. A molybdic acid assay may be used to determine the concentration of soluble silicate present in an aliquot of the buffer over time course of the assay. As shown in the examples, the composition may be diluted in 10 mM HEPES buffer and adjusted to pH 6.7-7.0. An exemplary molybdic acid assay employs 100 µL of the test solution or standard (prepared from Sigma Aldrich Si ICP standard, 1000 mg/L) and 200 µL molybdic acid colouring solution (0.6105 g NH₄Mo₇ 4H₂0, 15 mL 0.5 N H₂SO₄, 85 mL H₂O). The assay solution is transferred to a well plate and mixed for 10 minutes. After the incubation, the absorbance (405 nm) can be measured and the concentration of soluble silicic acid determined using a standard curve. By way of example, a "poorly condensed" polymeric silicate composition will be resorbable, for example as determined in an *in vitro* dissolution assay in which at least 25% of the composition, and more preferably at least 30%, and more preferably at least 35%, and more preferably at least 40% and more preferably at least 50% of the composition dissolves in 24 hours in HEPES buffer.

The stabilised silicate materials used in the present invention are metastable, that is the compositions possess a stability that is fit for the purpose of shelf-life of their intended use, e.g. as antiperspirants, and do not grow to any significant extent to form gels until they are applied to a subject. However, the application of the antiperspirant composition to a subject causes a pH shift that induces growth of the polysilicic acids to form a gel thereby providing an antiperspirant active ingredient through the formation of the gel in the sweat pores of the subject. By way of illustration, it is preferred that the silicate compositions are stable for more than 6 months, preferably 12 months or more, and more preferably 24 months or more. Thus, the polysilicic acids may be produced by partial condensation of silicic acid (or silicate) molecules. These materials are metastable as discreet, non-aggregated polysilicic acids and undergo a process of growth, leading to the formation of a gel, when exposed to a physiological trigger. This physiological trigger will typically be a change in pH, e.g. as occurs when the silicate composition are applied to the skin of a subject, but it will be clear to those skilled in the art that other triggers such as, but not limited to, change in the ionic strength, salt composition, increase in temperature, or exposure to endogenously produced molecules (e.g. lactic acid) might also be employed.

Conveniently, the progress of growth towards gel formation, and hence the stability of the silicate compositions, may be determined by measuring the viscosity of the compositions. In the experiments described herein, a viscosity above 8.0 mPa.s at 25°C corresponds to a fully formed gel. Preferably, the stabilised silicate compositions used in the present invention have a viscosity measured at 25°C of less than 4.0 mPa.s, more preferably less than 3.0 mPa.s and still more preferably less than 2.0 mPa.s. As is well known in the art, viscosity may be measured using a low frequency vibration method, for example using a SV-10 Vibro Viscometer (from A&D Ltd, Japan), in which two gold plated paddles are in a tuning fork arrangement at 30Hz. The oscillation amplitude depends on the viscosity of the material, which is measured in real time. It is preferred that the measurements are made on freshly prepared samples.

The compositions described herein are produced through a process of poly condensation of orthosilicic acid. This process of condensation is incomplete and produces materials that are distinct from silicon dioxide (SiO₂) and that can be defined by the following general compositional formula: [SiOₓ(OH)₄₋₂ₓ]ₙ where 0<x<2.

### Methods of producing stabilised silicate compositions

The present invention is based on a method for producing a method for producing stabilised polysilicic acids, the method comprising:
(a) preparing an alkaline silicate solution having a pH ≥ 9.5;
(b) optionally adding a growth retardant to the alkaline silicate solution;
(c) lowering the pH to ≤ 4.0 by adding an acid to form a composition comprising polysilicic acids;
(d) optionally adding a multivalent cation and/or a growth retardant;
(e) raising the pH of the composition to a physiological acceptable pH by adding a base, thereby forming the stabilised composition comprising polysilicic acids;
(f) optionally adding a growth retardant capable of increasing the stability of the composition, wherein the growth retardant is a carboxylic acid, an amino acid, an inorganic anion, a polyol, saccharide or a quaternary ammonium cation; and,
(g) optionally adding a non-aqueous solvent capable of increasing the stability of the composition; and
(h) incorporating the polysilicic acid composition into an antiperspirant formulation or a cosmetic formulation;
wherein the polysilicic acid is present in the form of polysilicic acid nanoparticles or clusters having mean diameters of 20 nm or less.

It will be apparent to those skilled in the art that it may be possible to reorder some of the steps of the above method and/or for some of the steps to take place simultaneously. Others of the steps are optional as indicated above and explained further below.

In the work leading to the present invention, the inventors found that a number of factors contribute to the stability of the silicate compositions including the rate at which the pH of the alkaline silicate solution is lowered, the inclusion of compounds found to work as growth retardants, the addition of multivalent cations and/or the addition of a non-aqueous solvent. Accordingly, the methods of the present invention may employ these approaches, alone or in any combination, to produce silicate compositions having sufficient stability for use, e.g. as antiperspirants.

Of these factors, the experiments below demonstrate that the rate at which the pH of the alkaline silicate solution is lowered in step (c) has a significant effect on the stability of the resulting polysilicic acid compositions. Preferably, the pH is lowered over a period of less than 60 seconds, more preferably less than 30 seconds, more preferably less that 10 seconds, or most preferably less that 5 seconds.

It will be clear to those skilled in the art that glass electrodes can be poisoned by the presence of polysilicic acids (i.e. clusters, colloids etc.) thus giving erroneous pH results. Therefore, great care must be taken when performing these measurements and the pH results should be confirmed using high quality pH strips as done in the work described herein.

In step (a), it is preferred that the concentration of the alkaline silicate solution is between 0.05 M and 1.0 M, and more preferably is between 0.1 M and 1.0 M. The use of pHs that are higher than 9.5 is also preferred in order to maintain the solubility of the silicates, and preferably in step(a) the pH of the alkaline silicate solution is about pH 10.5 or above, and still more preferably is about pH 11.5 or above.

The present invention is also based on the finding that including at least one growth retardant in the silicate solution before the pH is lowered helps to promote the stability of the compositions. The skilled person can carry out routine tests to determine which growth retardants work best in any given situation and it is possible to employ combinations of more than one different growth retardant, e.g. two, three, four or five or more growth retardants, e.g. by adding them in step (b). By way of example, growth retardants include carboxylic acids, including polycarboxylic acids such as polyacrylic acid, amino acids, inorganic anions, polyols such as a polyalkylene glycol and/or a quaternary ammonium ion, such as choline. Growth retardants are generally added at a concentration between 0.01 M and 3.0 M, and more preferably between 0.1 M and 1.5 M.

In embodiments of the present invention that use carboxylic acids as growth retardants, the carboxylic acid may be a C₂₋₁₀ carboxylic acid, for example a dicarboxylic acid such as oxalic acid, malonic acid, glutaric acid, tartaric acid, succinic acid, adipic acid or pimelic acid, or ionised forms thereof (i.e., the corresponding carboxylate), such as adipate. Or for example a monocarboxylic acid, such as gluconic acid. Further examples of growth retardants are dicarboxylic acids, which may be represented by the formula HOOC-R₁-COOH (or an ionised form thereof), where Ri is an optionally substituted C₁₋₁₀ alkyl, C₁₋₁₀ alkenyl or C₁₋₁₀ alkynyl group. In general, the use of carboxylic acids in which R₁ is a C₁₋₁₀ alkyl group, and more preferably is a C₂₋₆ alkyl group, is preferred. Preferred optional substituents of the Ri group include one or more hydroxyl groups, for example as present in malic acid. In preferred embodiments, the R₁ group is a straight chain alkyl group. A more preferred group of carboxylic acids include adipic acid (or adipate), glutaric acid (or glutarate), pimelic acid (or pimelate), succinic acid (or succinate), and malic acid (or malate). Whether the carboxylic acid is present as the acid or is partially or completely ionised and present in the form of a carboxylate anion will depend on a range of factors such as the pH at which the material is produced and/or recovered, the use of post-production treatment or formulation steps and how the carboxylic acid becomes incorporated into the stabilised polysilicic acid composition. For the avoidance of doubt, the use of carboxylic acid growth retardants in accordance with the present invention covers all of these possibilities, i.e. the growth retardant present as a carboxylic acid, in a non-ionised form, in a partially ionised form (e.g., if the growth retardant is a dicarboxylic acid) or completely ionised as a carboxylate ion, and mixtures thereof.

Examples of suitable amino acid growth retardants include aspartic acid. Examples of growth retardants that are polyols, i.e. multiple hydroxylated alcohol, include a monomeric polyol, such as glycerol, ethylene glycol, xylitol, propylene glycol, or a polyalkylene glycol, such as polyethylene glycol or polypropylene glycol. Examples of growth retardants that are sugars (saccharides) include monomeric, dimeric, trimeric and polymeric sugars, such as glucose, fructose, mannose, sucrose, threitol, erythritol, sorbitol, mannitol, galactitol or adonitol.

In the present invention, the polymeric silicate compositions include a growth retardant that is a polyalkylene glycol. Polyalkylene glycols are a family of polyether compounds that include polyethylene glycol (PEG) and polypropylene glycol. In some embodiments, it is possible to employ combinations of more than one different polyalkylene glycols, e.g. two, three, four or five or more sugars or polyalkylene glycols, e.g. by adding them in step (a) and/or (b). Polyalkylene glycol growth retardants are generally added at a concentration between 0.01 M and 3.0 M, and more preferably between 0.03 and 2.0 M, and most preferably between 0.1 M and 1.5 M. The skilled person can carry out routine tests to determine which combinations of sugars and/or polyalkylene glycols work best in any given situation.

Without wishing to be bound by any particular theory, the present inventors do not believe that these materials act as ligands in a conventional sense in having a strong interaction involving the donation of one or more electron pairs between a ligand (donor) and a central atom (acceptor) to form a coordination complex, but rather have a weaker interaction that is nonetheless capable of stabilising the silicate compositions in the form of polysilicic acids.

In step (c), it is preferred that the pH of the composition is lowered to a pH ≤ 1.5 in order to inhibit the polysilicic acids growth that would otherwise occur below a pH of about 9.0 and lead to the uncontrolled formation of silicate gels.

In some embodiments, the polysilicic acids may be contacted with multivalent cations, such as Ca²⁺, Mg²⁺, Cu²⁺, Fe³⁺ and/or Zn²⁺ as the inventors have found that this helps to stabilise the compositions. Without wishing to be bound by any particular theory, the present inventors believe that the cations coat the polysilicic acids via interaction with free silanol groups (-OH) present in the materials. By way of guidance, it is preferred that the multivalent cation is added to provide a final concentration between 0.01 M and 1.0 M and more preferably the multivalent cation is added to provide a final concentration between 0.05 M and 0.5 M. The addition of Cu²⁺ or Ag⁺ to the stabilised polysilicic acid compositions used in the present invention has the further advantage of limiting bacterial growth (herein illustrated with an *E. coli* model). The limitation of bacterial growth is particularly advantageous since it is associated with the release of axilla odour caused by the action of skin flora bacteria breaking down lipid components of sweat. As noted above, the inclusion of Al³⁺ is generally not preferred.

In step (e), once the growth of polysilicic acids has been inhibited, it is preferred that the pH of the composition is raised to a physiological pH to adapt the formulation so that it can be used in antiperspirant compositions, preferably to a pH between 3.0 and 5.0, more preferably to a pH between 2.5 and 4.5, more preferably to a pH between 3.0 and 4.5, and more preferably to a pH of between 3.5 and 4.0. Conveniently, this may be done by adding a base, such as sodium hydroxide or sodium carbonate.

The present inventors also surprisingly found that polysilicic acid compositions used in the present invention may further stabilised by adding a non-aqueous solvent, such as an alcohol. A preferred example of an alcohol is ethanol. By way of illustration, the non-aqueous solvent may be added between 5 and 70% v/v, or between 10 and 60% v/v, or between 10 and 50% v/v, or between 20 and 50% v/v or between 10 and 20% v/v. Furthermore, in some cases the present inventors found that the combination of polyols (such as a polyalkylene glycol) with alcohol was particularly effective for stabilising the compositions.

### Antiperspirant compositions

Antiperspirant composition of the present invention may be formulated according to any approach known in the art, for example for delivery by an aerosol device, a pump-dispenser bottle, a roll-on, a device equipped with a perforated wall, or a stick. As the antiperspirant active is provided by the stabilised silicates preferably the composition does not comprise aluminium or zirconium salts as additional antiperspirant active.

In addition, the antiperspirant compositions used in the present invention may comprise one more of an additional antiperspirant active agent, a deodorant, a volatile and non-volatile oil, silicone and hydrocarbon-based emollient oils, a suspension agent, an organic powder, a water-immiscible organic liquid phase and at least one agent for structuring said phase and/or a perfume or fragrance, perfume solubilising agent or wash off agent.

In addition, a composition comprising the stabilised polysilicic acid particles used in the present invention may be incorporated into an antiperspirant composition or a cosmetic formulation comprising a polyalkylene glycol, such as PEG. Polyalkylene glycols, such as PEG, are especially well suited for topical delivery of silicate as it readily forms a cream or an ointment well suited for formulation in an antiperspirant composition and is available in a range of different molecular weights, allowing the tailoring of viscosity and other physical parameters that may desirable in the final formulation. It will be obvious to those in the art that topical application delivery may also be achieved using non-PEG based ointments. In this case, upon initial stabilisation with PEG as described herein, the silicates are incorporated in a non-PEG based ointment, e.g. a PEG stabilised nanosilicate composition incorporated in a further, different vehicle such as hydroxyethyl cellulose.

These antiperspirant compositions or cosmetic formulations compositions can be in the form of creams, lotions, gels, suspensions, dispersions, microemulsions, nanodispersions, microspheres, hydro gels, emulsions (oil-in-water and water-in-oil, as well as multiple emulsions) and multilaminar gels and the like, see, for example, The Chemistry and Manufacture of Cosmetics, Schlossman et al., 1998. The compositions may be formulated as aqueous or silicone compositions or may be formulated as emulsions of one or more oil phases in an aqueous continuous phase (or an aqueous phase in an oil phase). The type of carrier utilized in the present invention depends on the type of product form desired for the topical composition. The carrier can be solid, semi-solid or liquid. Suitable carriers are liquid or semi-solid, such as creams, lotions, gels, sticks, ointments, pastes, sprays and mousses. Specifically, the carrier is in the form of a cream, an ointment, a lotion or a gel, more specifically one which has a sufficient thickness or yield point to prevent the particles from sedimenting. The carrier can itself be inert or it can possess benefits of its own. The carrier should also be physically and chemically compatible with the stabilised polymeric silicate compositions or other ingredients formulated in the carrier. Examples of carriers include water, hydroxyethyl cellulose, propylene glycol, butylene glycol and polyethylene glycol, or a combination thereof.

Examples of perfumes include perfume oils, deo-perfumes as disclosed in EP 0 545 556 A or the perfumes disclosed in US 2014/179748. The amounts of perfume added to antiperspirant compositions are preferably up to 5% by weight, more preferably from 0.1 % to 3.5% by weight, and more preferably from 0.5% to 2.5% by weight. The fragrance or perfume may also be added in an encapsulated form, release being triggered post-application by hydrolysis or shear on the surface of the human body.

### Medical uses

In addition to cosmetic applications of the present invention, e.g. in personal care compositions, the present invention has uses for the treatment of medical conditions characterised by excessive sweating. Hyperhidrosis is a medical condition in which a person sweats excessively and unpredictably, often without normal temperature or environmental triggers of normal sweating. Subjects suffering from hyperhidrosis may have overactive sweat glands and the conditions leads to uncontrollable sweating, causing significant physical and emotional discomfort. As is known in the art, primary or focal hyperhidrosis is characterised by excessive sweating affecting the hands, feet and armpits. In situations in which excessive sweating is the result of another medical condition, it is called secondary hyperhidrosis and in such cases the sweating may affect any part of the body. Medical conditions that lead to secondary hyperhidrosis include acromegaly, anxiety disorders, cancer, including leukaemia and non-Hodgkin's lymphoma, carcinoid syndrome, endocarditis, heart attack, hyperthyroidism, substance abuse, obesity, diabetes, heart disease, HIV/AIDS, hyperthyroidism, lung disease, medications, such as beta blockers and tricyclic antidepressants, menopause, Parkinson's disease, pheochromocytoma, spinal cord injury, stroke, stress, tuberculosis, fever or infection.

### Experimental

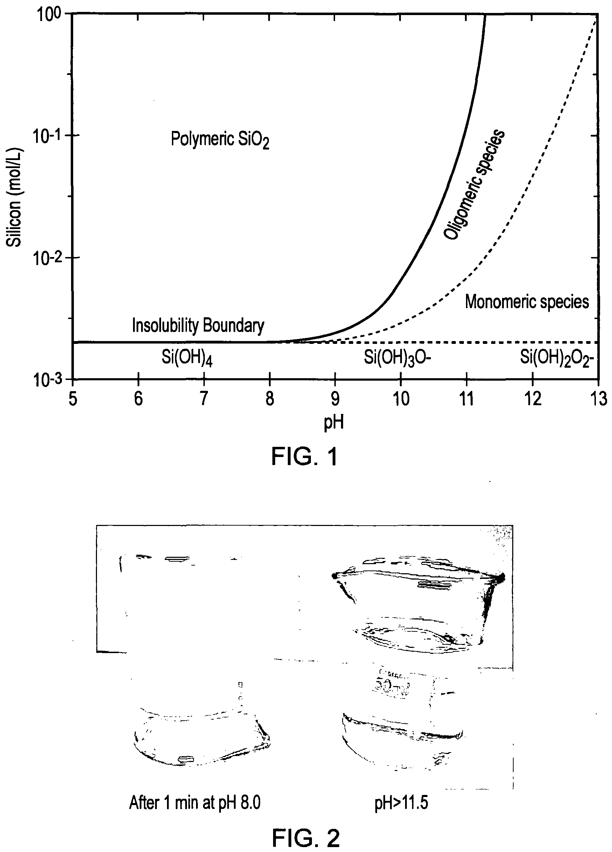

Silicate (or silicic acid) is very biocompatible and can form strong, clear gels. Its use as an antiperspirant has not been documented, however. This is not surprising, since the chemistry of silicate does not make it an obvious antiperspirant candidate as it behaves in an opposite fashion to aluminium, remaining soluble only above pH 10.5-11 (Figure 1). Such caustic solutions cannot be directly applied to the skin and if the pH of the silicate solution were lowered to more tolerable levels (e.g. to pH 8.0), then growth would proceed rapidly, i.e. within the formulated antiperspirant material rather than in the sweat pore, as shown in Figure 2. Moreover, even if the solubility of silicates could be extended to lower pHs, the problem remains that the buffering capacity of skin is far lower for moderately alkaline solutions than it is for acidic solutions such as those of aluminium antiperspirants.

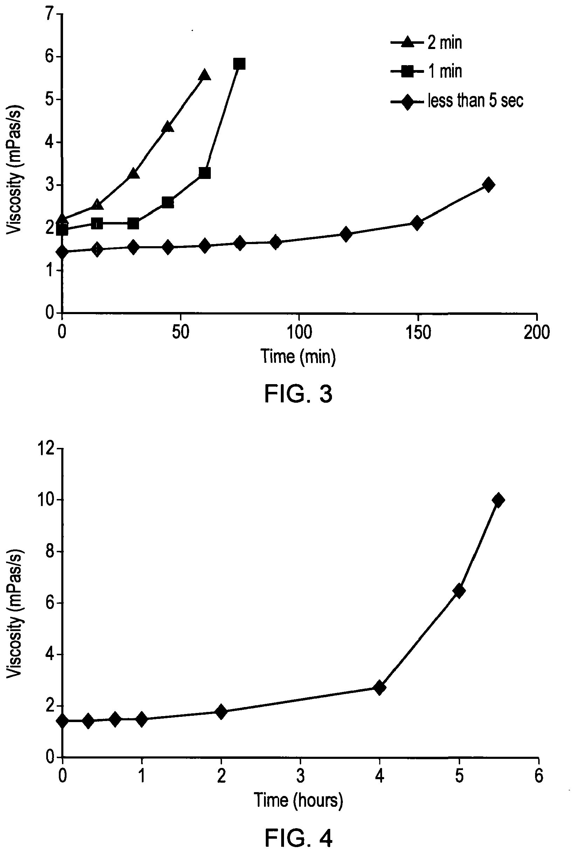

The present invention is based on the finding that it is possible to produce silicate compositions in which the silicate is present in the form of polysilicic acids, rather than monomeric silicic acid, and that the silicate compositions are sufficiently stable to be employed as antiperspirants. A first key observation was that when the pH of silicate solutions is lowered sufficiently, preferably below pH 7.0 (e.g. pH 2.5 or 3.0 to 5.0, and more preferably about 4.0), the growth rate slows down dramatically from seconds to hours. The second key finding is that this phenomenon is affected by the rate at which pH is lowered (see Figure 3). These actions are sufficient to prevent growth and gel formation for around 8 hours (see Figure 4).

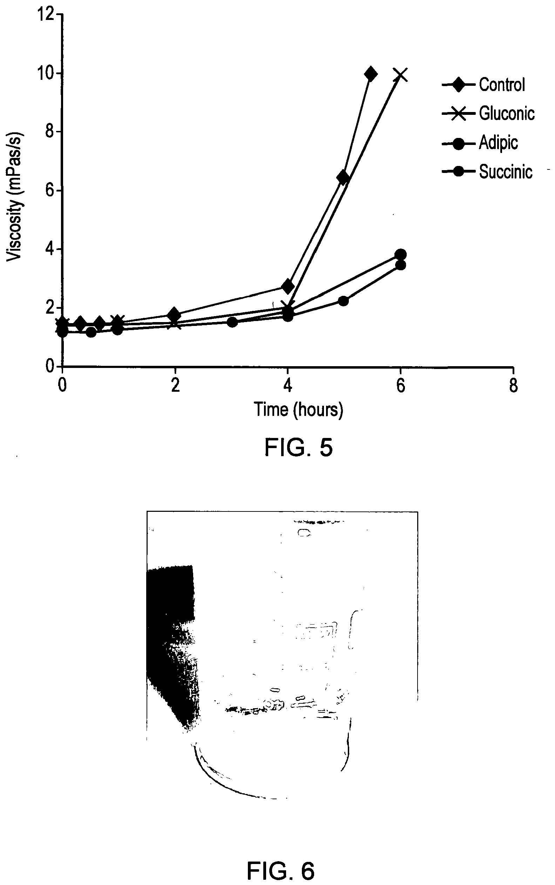

The present inventors then found that the rate of growth of polysilicic acids can be further reduced by addition of further growth retardants (Table 1). Although, the addition of these growth retardants does not lead to the prevention of polymerisation, some growth retardants further reduce the rate of growth (Figure 5) to an extent where an optimised silicate composition may have a stability sufficient for formulation in an antiperspirant composition.

**Table 1. Compounds tested as growth retardants. A gel was formed within 24 hours for all combinations tested. The growth retardants were added to a 0.5 M silicate solution, prior to lowering to pH 4.**

| Type of Growth Retardant | Compound | Concentration tested (M) |
|---|---|---|
| Carboxylic Acid | Oxalic Acid | 0.1 |
| | Malonic Acid | 0.1 |
| | Gluconic Acid | 0.05, 0.1, 0.5, 1.5 |
| | Tartaric Acid | 0.1 |
| | Succinic Acid | 0.05, 0.1, 0.5, |
| | Adipic Acid | 0.05, 0.1 |
| | Pimelic Acid | 0.1 |
| Amino Acids | Aspartic Acid | 0.1 |
| Choline | Choline | 0.05, 0.1, 0.5, |
| Inorganic | Phosphate | 0.1 |
| Polyols | Glycerol | 1.5 |
| | Xylitol | 0.1 |

Surprisingly, the present inventors found that polyols were particularly effective in suppressing growth when added together with a non ionic solvent. In the following experiments an alcohol (ethanol) was chosen as the non ionic-solvent. Table 2 shows the combinations which were most effective at suppressing growth. The glycerol/ethanol combination is of particular interest as it remained stable for more than seven days.

Importantly, the stabilisation observed in these experiments occurs between about pH 2.5 (or 3.0) and 5.0 (e.g. at about pH 4) and once the pH of the silicate solutions is raised to pH 6.0 (as in the sweat duct) the growth process leads to the relatively rapid formation of a gel (Figure 6) suitable for use as an antiperspirant active.

**Table 2. Combination of polyols with ethanol that supressed growth for more than five days. The polyols were added to the silicate solution prior to lowering to pH 4 and the ethanol was added after the pH adjustment.**

| [Si] mol/L | Stabilizer | [Stabiliser] mol/L | Ethanol % (V/V) |
|---|---|---|---|
| 0.3 | Glycerol | 1.8 | 50 |
| 0.4 | Xylitol | 1.2 | 20 |

| | | | |
|---|---|---|---|
| Note: viscosity measurements were not possible as these would lead to a loss of ethanol and subsequent growth to form a gel. | | | |

In addition to these investigations, the present inventors also found that complex polyols, such as sucrose, are able to be used as growth retardants, and initial experiments show that they are capable of supressing growth for weeks, even in the absence of ethanol. Importantly, these silicate solutions still undergo growth upon a pH shift to pH 6.

The following examples illustrate the preparation of stabilised silicate compositions according to the present invention.

### Example 1

A 0.5 M silicate solution at pH ∼ 11.5 was prepared. Next the pH was lowered to pH < 1.5 by adding an appropriate volume of 37% HCl all at once. NaOH (0.1-0.5 M) was added to adjust the pH to 3.5-4.0.

### Example 2

A 0.5 M silicate solution at pH ∼ 11.5 was prepared. Next the pH was lowered to pH < 1.5 by adding an appropriate volume of 37% HCl all at once. NaOH (0.1- 0.5 M) was added to adjust the pH to 3.5-4.0. Finally, X % ethanol was added, where X= 10, 20, 30 or 50%.

### Example 3

A 0.5 M silicate solution at pH ∼ 11.5 was prepared. Then sucrose was added to obtain a final concentration of 1.5 M. Next the pH was lowered to pH < 1.5 by adding an appropriate volume of 37% HCl all at once. Then CaCl₂ was added to obtain a final concentration of Y. NaOH (0.1-0.5 M) was added to adjust the pH to 3.5-4.0. Y= 0.05, 0.1 or 0.25 M CaCl₂.

### Example 4

A 0.5 M silicate solution at pH ∼ 11.5 was prepared. Then sucrose was added to obtain a final concentration of 1.5 M. Next the pH was lowered to pH < 1.5 by adding an appropriate volume of 37% HCl all at once. Then CaCl₂ was added to obtain a final concentration of 0.1M. Sodium carbonate (0.1-0.5 M) was added to adjust the pH to 3.5-4.0.

### Example 5

A silicate solution at pH ∼ 11.5 was prepared. The growth retardant (refer to table below; applicable to all retardants except PEG and polyacrylic acid) was then dissolved in this solution. Next the pH was lowered to pH <1.5 by adding an appropriate volume of 37% HCl all at once. NaOH (0.1- 0.5 M) was added to adjust the pH to 3.5-4.0.

### Example 6

A silicate solution at pH ∼ 11.5 was prepared. Next the pH was lowered to pH <1.5 by adding an appropriate volume of 37% HCl all at once. NaOH (0.1- 0.5 M) was added to adjust the pH to 3.5-4.0. The growth retardant was then dissolved in this solution (see the table below).

### Example 7

Any material from examples 1 to 6 was incorporated into a PEG cream according to the following procedure. PEG 3350 (5.25 g) was melted and sodium hydroxide was added to ensure the pH of the cream, once formed, is above pH 3. PEG 200-stabilized polysilicic acids (2.3 g of suspension) are mixed with PEG 400 (6.15 g) at 65-70°C and added to the PEG melt. The resulting mixture was homogenised and allowed to cool to room temperature.

### Example 8

Any material from Examples 1 to 6 was incorporated into a PEG cream according to the following procedure. PEG 3350 (5.25 g) was melted and sodium hydroxide was added to ensure the pH of the cream, once formed, is above pH 3. PEG 200-stabilized polysilicic acids (2.3 g of suspension) are mixed with PEG 400 (6.15 g) at room temperature and added to the PEG melt. The resulting mixture was homogenised and allowed to cool to room temperature.

### Example 9

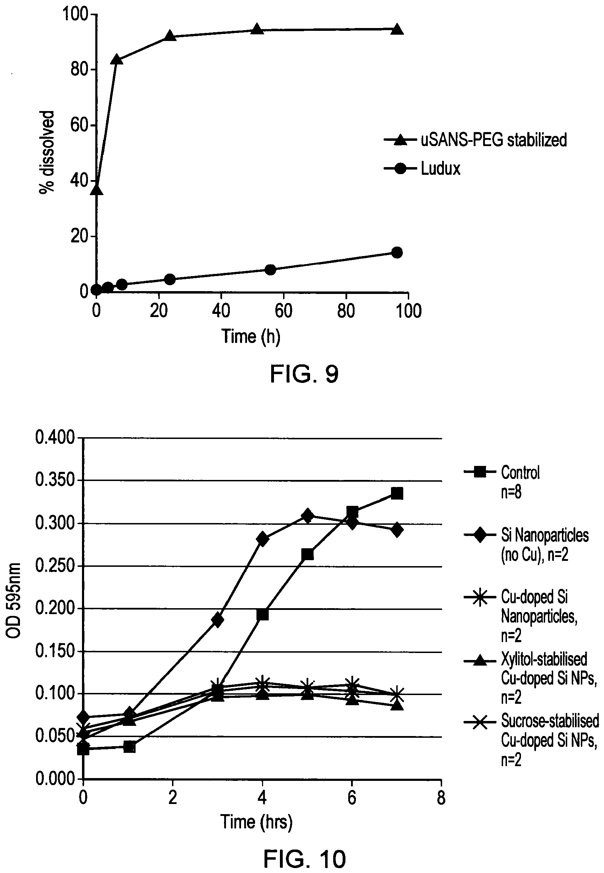

The dissolution of the stabilised polysilicic acid compositions of the present invention was compared to the dissolution of a commercially available fully condensed form of colloidal silica as described in US 2007/148113 using Ludox SM30® (http://www.sigmaaldrich.com/catalog/product/aldrich/420794?lang= en&region=GB). Figure 9 shows that the stabilised polysilicic acid compositions of the present invention are labile and substantially completely dissolve, whereas dissolution of the colloidal silica is less than 20%.

### Example 10

The hydrodynamic particle size of stabilised polysilicic acid compositions of the present invention stabilised with 1.5 M sucrose was determined 1 hour after synthesis, showing that the particle sizes ranged from about 1nm to about 3nm.

### Example 11

Using the process described herein, the size tailorability upon dropping the pH, of small particles (<5nm; typically <3.5 nm) was determined. However, larger particle sizes can be achieved by raising the pH. Usefully, the rate of growth can be determined by selecting the appropriate pH and concentrations. Figure 7 shows how a slower growth rate can be achieved by only raising the pH to 4. As stated above, size growth can be arrested by adding a stabiliser (e.g. PEG, Figure 8) or diluting the suspension.

### Example 12

The addition of Cu²⁺ to the stabilised polysilicic acid compositions of the present invention has the further advantage of limiting bacterial growth (herein illustrated with an E coli model). The limitation of bacterial growth is particularly advantageous since it is associated with the release of axilla odour caused by the action of skin flora bacteria breaking down lipid components of sweat. Accordingly, copper doped polysilicic acids were synthesized using 0.5 M silicate solution at pH ∼ 11.5. Next the pH was lowered to pH < 1.5 by adding an appropriate volume of 37% HCl all at once. CuCl₂.2H₂O was added to achieve a final concentration of 25 mM Cu. NaOH (0.1-0.5 M) was added to adjust the pH to 3.5-4.0.

The antimicrobial action of stabilised polysilicic acid compositions of the present invention was tested. Copper loaded silicate polymers showed antimicrobial activity, but growth retardants did not impact negatively in the bacterial activity of copper. There was not a remarkable difference between stabilised and non-stabilised materials (Figure 10). In practice, stabilisation would allow greater copper-loaded silicate concentrations and no impact of the stabiliser on efficacy.

### Example 13

The antiperspirant efficacy of stabilised polysilicic acids (SiA) was compared to that of aluminum chlorohydrate (ACH). ACH is used as a topical antiperspirant or body deodorant by reducing perspiration, limiting the sweat available for skin bacteria to decompose into odorous products. It is the current "gold standard" antiperspirant. Stabilised polysilicic acids were prepared for testing as follows. 8ml of 6.25M sodium silicate solution were diluted in 50ml of UHP H₂O. Next the pH was dropped to <1.5 by rapidly adding 4ml of 37% HCl under constant stirring. The final pH was adjusted to 3.5±0.5 (pH-indicator strips pH 0-6, BDH 31505), using firstly 5M NaOH (ca. 0.5ml) and then 0.5M NaOH (ca. 2ml). 30ml of ethanol were added and final volume was adjusted to 100ml by adding UHP H₂O.

| **Ingredient** | **"ACH"** | **"SiA"** |
|---|---|---|
| Aluminum chlorohydrate | 10% | - |
| Polysilicic acids (as [Si]) | - | 0.5M |
| Ethanol | - | 30% (v/v) |
| H₂O | ad 100 % | ad 70% |
| Final pH readout | 4.0 ± 0.5 | 4.0 ± 0.5 |

### Example 14

### Antiperspirant efficacy test

### Method:

A gravimetric sweat test was conducted using cotton pads to collect axillary sweat. Briefly, 24 subjects (12 female + 12 male) having refrained from using antiperspirants for more than two weeks were included. A single axillary application of 500mg solution was performed blinded and right-left randomized for both test solutions ("ACH" vs. "SiA"). Six hours after application cotton pads were placed in both axillae and sweating was induced by entering a sauna at 75°C and 30% relative humidity. Sweat was collected for 15 minutes and estimated as weight increase of the pads.

### Results:

The amount of axillary sweat was normalized to baseline sweat rates obtained from a sauna test under identical conditions before product application (=100%) to calculate the relative sweat reduction. As shown in the table below, the polysilicic acid composition of the present invention was as effective as ACH under the chosen test conditions, without the risks known to be associated with aluminium compositions.

| sample | baseline [g] | after treatment [g] | relative reduction [%] | significance p |
|---|---|---|---|---|
| "ACH" | 0.75 ± 0.45 | 0.35 ± 0.24 | 53,3 | <0.001 |
| "SiA" | 0.74 ± 0.48 | 0.34 ± 0.29 | 54,1 | <0.001 |

**Table 3: Examples of combinations of reagents used in the production of the compositions of the present invention and ratios used are provided below.**

| [Silicon], M | Retardant | [retardant], M | Other components |
|---|---|---|---|
| 0.5 | Glutaric acid | 0.1 | |
| 0.5 | Adipic acid | 0.1 | |
| 0.5 | Adipic acid | 0.1 | + 20% Ethanol |
| 0.5 | Pimelic acid | 0.1 | |
| 0.5 | Oxalic acid | 0.1 | |
| 0.5 | Malonic acid | 0.1 | |
| 0.5 | Succinic acid | 0.05-0.5 | |
| 0.5 | Gluconic acid | 0.05-1.5 | |
| 0.5 | Xylitol | 0.1-3.0 | |
| 0.5 | Xylitol | 1.5 | +20% Ethanol |
| 0.3 | Xylitol | 0.9-1.2 | +50% Ethanol |
| 0.5 | Glycerol | 1.5-3.0 | |
| 0.5 | Glycerol | 1.5-3.0 | |
| 0.5 | Sucrose | 0.5-3.0 | |
| 0.5 | Glucose | 1.5 | |
| 0.5 | Fructose | 1.5 | |
| 0.5 | Maltose | 0.5-1.5 | |
| 0.5 | Polyacrylic acid | 0.5-1.0 * | |
| 0.5 | Polyethylene glycol | 0.5-1.0 * | |

| | | | |
|---|---|---|---|
| * refers to the concentration of the monomer group within the polymer | | | |

### References:

1. Markey, Botulinum A exotoxin in cosmetic dermatology. Clinical and Experimental Dermatology, 25(3): 173-175, 2000.
2. Tomljenovic, Aluminum and Alzheimer's Disease: After a Century of Controversy, Is there a Plausible Link? Journal of Alzheimers Disease, 23(4): 567-598, 2011.
3. Darbre, Aluminium, antiperspirants and breast cancer. Journal of Inorganic Biochemistry, 99(9): 1912-1919, 2005.
4. Jugdaohsingh et al, Is there a biochemical role for silicon?, in Metal Ions in Biology and Medicine, Vol. 10, P. Collery, et al., Editors. 2008, John Libbey Eurotext: Montrouge. pages 45-55.
   US 7,303,767.
   US 5,468,473.
   US 2007/148113

## Claims

1. A device for delivering an antiperspirant composition comprising a stabilised silicate composition which comprises polysilicic acids, wherein the antiperspirant composition is as obtainable by a method comprising providing an alkaline silicate solution having a pH ≥ 9.5 and lowering the pH to between 3.0 and 5.0 prior to application to a subject, wherein the application of the antiperspirant composition to the subject causes a pH shift in the composition that induces growth of the polysilicic acids to form a gel thereby providing an antiperspirant active ingredient, wherein the polysilicic acid is present in the form of polysilicic acid nanoparticles having mean diameters of 20 nm or less, and wherein the device is selected from an aerosol device, a pump-dispenser bottle, a roll-on, a device equipped with a perforated wall, or a wand (stick);
wherein the pH is lowered over a period of less than 60 seconds; and/or
wherein the polysilicic acid composition is stabilised by a growth retardant, wherein the growth retardant is a carboxylic acid, an amino acid, an inorganic anion, a polyol, saccharide or a quaternary ammonium cation; and/or
wherein the polysilicic acid composition is stabilised by a multivalent cation and/or by a non-aqueous solvent.

2. A non-therapeutic cosmetic process for treating human body odour using an antiperspirant composition comprising a stabilised silicate composition which comprises polysilicic acids, wherein the antiperspirant composition is as obtainable by a method comprising providing an alkaline silicate solution having a pH ≥ 9.5 and lowering the pH to between 3.0 and 5.0 prior to application to a subject, wherein the application of the antiperspirant composition to the subject causes a pH shift in the composition that induces growth of the polysilicic acids to form a gel thereby providing an antiperspirant active ingredient, wherein the polysilicic acid is present in the form of polysilicic acid nanoparticles having mean diameters of 20 nm or less;
wherein the pH is lowered over a period of less than 60 seconds; and/or
wherein the polysilicic acid composition is stabilised by a growth retardant, wherein the growth retardant is a carboxylic acid, an amino acid, an inorganic anion, a polyol, saccharide or a quaternary ammonium cation; and/or
wherein the polysilicic acid composition is stabilised by a multivalent cation and/or by a non-aqueous solvent.

3. An antiperspirant composition for use in a method of treating of a medical condition **characterised by** excessive perspiration using an antiperspirant composition comprising a stabilised silicate composition which comprises polysilicic acids wherein the antiperspirant composition is as obtainable by a method comprising providing an alkaline silicate solution having a pH ≥ 9.5 and lowering the pH to between 3.0 and 5.0 prior to application to a subject, wherein the application of the antiperspirant composition to the subject causes a pH shift in the composition that induces growth of the polysilicic acids to form a gel thereby providing an antiperspirant active ingredient, wherein the polysilicic acid is present in the form of polysilicic acid nanoparticles having mean diameters of 20 nm or less;
wherein the pH is lowered over a period of less than 60 seconds; and/or
wherein the polysilicic acid composition is stabilised by a growth retardant, wherein the growth retardant is a carboxylic acid, an amino acid, an inorganic anion, a polyol, saccharide or a quaternary ammonium cation; and/or
wherein the polysilicic acid composition is stabilised by a multivalent cation and/or by a non-aqueous solvent.

4. The device, non-therapeutic cosmetic process or the composition for use in a method of treatment of any one of claims 1 to 3, wherein the polysilicic acid composition is stabilised by a growth retardant, wherein the growth retardant is a carboxylic acid, an amino acid, an inorganic anion, a polyol, saccharide or a quaternary ammonium cation.

5. The device, non-therapeutic cosmetic process or the composition for use in a method of treatment of any one of claims 1 to 4, wherein the pH shift is to a pH of above 5 and below 8, preferentially 5.5 to 6.5, and more preferably to a pH of about 6.0.

6. The device, non-therapeutic cosmetic process or the composition for use in a method of treatment of any one of claims 1 to 5, wherein the pH shift occurs in skin pores and causes production of the silicate gel in the pores.

7. The device, non-therapeutic cosmetic process or the composition for use in a method of treatment of any one of claims 1 to 6, wherein the pH of the composition before application to the subject or optionally between pH 3.0 to 4.5, or optionally between pH 3.0 and 4.5, or optionally between pH 3.5 and 4.0.

8. The device, non-therapeutic cosmetic process or the composition for use in a method of treatment of any one of claims 1 to 7, wherein the stabilised silicate is stabilised by at least one growth retardant, and optionally by two, three, four or five growth retardants, the growth retardant(s) are selected from a carboxylic acid, an amino acid, an inorganic anion, a polyol, saccharide and a quaternary ammonium cation.

9. The device, non-therapeutic cosmetic process or the composition for use in a method of treatment of any one of claims 1 to 8, wherein the carboxylic acid is a C₂₋₁₀ carboxylic acid, and preferably a dicarboxylic acid such as oxalic acid, malonic acid, gluconic acid, glutaric acid, tartaric acid, succinic acid, adipic acid or pimelic acid, the amino acid is aspartic acid, a polyol such as a monomeric polyol, such as glycerol, ethylene glycol, xylitol, propylene glycol, or a polyalkylene glycol, such as polyethylene glycol or polypropylene glycol, or the polyol is a saccharide, such as glucose, fructose, mannose, sucrose, threitol, erythritol, sorbitol, dextran, mannitol, dextran, galactitol or adonitol, or the quaternary ammonium cation is choline.

10. The device, non-therapeutic cosmetic process or the composition for use in a method of treatment of any one of claims 1 to 9, wherein:
(a) the antiperspirant composition comprises one or more polyalkylene glycols; or
(b) the an antiperspirant composition comprises polyethylene glycol (PEG).

11. The device, non-therapeutic cosmetic process or the composition for use in a method of treatment of any one of claims 1 to 10, wherein the polysilicic acid particles are poorly condensed as determined in an *in vitro* dissolution assay in which at least 25%, or at least 30%, or at least 35%, or at least 40%, or at least 50% of the composition dissolves in 24 hours in HEPES buffer.

12. The device, non-therapeutic cosmetic process or the composition for use in a method of treatment of claim 10, wherein the *in vitro* dissolution assay is a molybdic acid assay for determining the soluble silicic acid fraction.

13. The device, non-therapeutic cosmetic process or the composition for use in a method of treatment of any one of claims 1 to 12, wherein the stabilised silicate composition comprises a multivalent cation such as Ca²⁺, Mg²⁺, Cu²⁺, Fe³⁺ and/or Zn²⁺, optionally wherein the multivalent cation is added to provide a final concentration between 0.01 M and 1.0 M, and preferably a final concentration between 0.05 M and 0.5 M.

14. The device, non-therapeutic cosmetic process or the composition for use in a method of treatment of any one of claims 1 to 13, wherein the stabilised silicate composition is stabilised by a non-aqueous solvent, such as an alcohol, optionally wherein the alcohol is ethanol and/or the non-aqueous solvent is added between 10 and 70% v/v.

15. The device, non-therapeutic cosmetic process or the composition for use in a method of treatment of any one of claims 1 to 14, wherein the composition comprises one or more of an additional antiperspirant active agent, a deodorant, a volatile or non-volatile oil, silicone and hydrocarbon-based emollient oils, a suspension agent, a perfume or fragrance, an organic powder and/or a water-immiscible organic liquid phase and at least one agent for structuring said phase.

16. The device, non-therapeutic cosmetic process or the composition for use in a method of treatment of any one of claims 1 to 15, wherein the composition does not comprise aluminium or zirconium salts.

17. The antiperspirant composition for use in a method of treatment of claims 3 to 16, wherein the medical condition is hyperhidrosis, optionally wherein the hyperhidrosis is primary or focal hyperhidrosis or secondary hyperhidrosis.

18. The antiperspirant composition for use in a method of treatment of claims 3 to 17, wherein the excessive perspiration is caused by acromegaly, anxiety disorders, cancer, including leukaemia and non-Hodgkin's lymphoma, carcinoid syndrome, endocarditis, heart attack, hyperthyroidism, substance abuse, obesity, diabetes, heart disease, HIV/AIDS, hyperthyroidism, lung disease, medications, such as beta blockers and tricyclic antidepressants, menopause, Parkinson's disease, pheochromocytoma, spinal cord injury, stroke, stress, tuberculosis, fever or infection.

19. A method for producing an antiperspirant composition which comprises stabilised polysilicic acids, the method comprising:
(a) preparing an alkaline silicate solution having a pH ≥ 9.5;
(b) optionally adding a growth retardant to the alkaline silicate solution;
(c) lowering the pH to ≤ 4.0 by adding an acid to form a composition comprising polysilicic acids;
(d) optionally adding a multivalent cation and/or a growth retardant;
(e) raising the pH of the composition to a physiological acceptable pH by adding a base, thereby forming the stabilised composition comprising polysilicic acids;
(f) optionally adding a growth retardant capable of increasing the stability of the composition, wherein the growth retardant is a carboxylic acid, an amino acid, an inorganic anion, a polyol, saccharide or a quaternary ammonium cation; and,
(g) optionally adding a non-aqueous solvent capable of increasing the stability of the composition; and
(h) incorporating the polysilicic acid composition into an antiperspirant formulation;
wherein the polysilicic acid is present in the form of polysilicic acid nanoparticles having mean diameters of 20 nm or less and the antiperspirant composition has a pH between 3.0 and 5.0.

20. The method of claim 19, wherein:
(i) in step (c) the pH is lowered over a period of less than 60 seconds, less than 30 seconds, less that 10 seconds, or less than 5 seconds; and/or
(ii) in step (c) the pH is lowered to a pH ≤ 3.0; and/or
(iii) the concentration of the silicate solution is between 0.05 M and 3.0 M, optionally wherein the concentration of the silicate solution is between 0.1 M and 1.5 M; and/or
(iv) the silicate composition is stable for 6 months or more, 12 months or more or 24 months or more; and/or.
(v) the polysilicic acid particles have a mean diameter less than 20 nm, optionally 10nm or less, optionally 5 nm or less and optionally between 1 and 3 nm; and/or
(vi) the stabilised silicate composition has a viscosity measured at 25°C of less than 4.0 mPa.s; and/or
(vii) in step(a) the pH of the alkaline silicate solution is about 11.5; and/or
(viii) at least one growth retardant is added in step (b), and optionally wherein two, three, four or five growth retardants are added in step (b); and/or
(ix) the growth retardant is a carboxylic acid, an amino acid, an inorganic anion, a polyol (such as a polyalkylene glycol), saccharide and/or quaternary ammonium cation, and optionally wherein the carboxylic acid is a C₂₋₁₀ carboxylic acid, and preferably a dicarboxylic acid such as oxalic acid, malonic acid, gluconic acid, glutaric acid, tartaric acid, succinic acid, adipic acid or pimelic acid, the amino acid is aspartic acid, a polyol such as a monomeric polyol, such as glycerol, ethylene glycol, xylitol, propylene glycol, or a polyalkylene glycol, such as polyethylene glycol or polypropylene glycol, or the polyol is a saccharide, such as glucose, fructose, mannose, sucrose, threitol, erythritol, sorbitol, dextran, mannitol, galactitol or adonitol, or the quaternary ammonium cation is choline; and/or
(x) in step (c) the pH of the composition is lowered to a pH ≤ 1.5; and/or
(xi) the multivalent cation is Ca²⁺, Mg²⁺, Fe³⁺, Cu²⁺ and/or Zn²⁺; and/or
(xii) the multivalent cation is added to provide a final concentration between 0.01 M and 1.0 M, and optionally wherein the multivalent cation is added to provide a final concentration between 0.05 M and 0.5 M; and/or
(xiii) in step (e) the pH of the composition is raised to a pH between 3.0 and 5.0, and optionally wherein in step (e) the pH of the composition is raised to a pH of between 3.5 and 4.0, optionally wherein the pH is raised using a base which is sodium hydroxide or sodium carbonate; and/or
(xiv) the non-aqueous solvent is an alcohol, and optionally wherein the alcohol is ethanol and/or the non-aqueous solvent is added between 10 and 70% v/v.

21. The method of claim 19 or claim 20, wherein the method comprises the further step of formulating the stabilised silicate composition with one or more additional components to produce an antiperspirant composition.

22. The method of claim 21, wherein the stabilised polysilicic acid particles are incorporated into a an antiperspirant composition or a cosmetic formulation comprising one or more polyalkylene glycols, and optionally wherein the stabilised polysilicic acid particles are incorporated into a an antiperspirant composition or a cosmetic formulation comprising polyethylene glycol (PEG).

23. The method of claim 21 or claim 22, wherein the additional components comprise one more of an additional antiperspirant active agent, a deodorant, an oil chosen from volatile and non-volatile, silicone and hydrocarbon-based emollient oils, a suspension agent, an organic powder and/or a water-immiscible organic liquid phase and at least one agent for structuring said phase.

## Patentansprüche

1. Vorrichtung zur Zufuhr einer Antitranspirantzusammensetzung, umfassend eine stabilisierte Silicatzusammensetzung, die Polykieselsäuren umfasst, wobei die Antitranspirantzusammensetzung durch ein Verfahren erhältlich ist, das das Bereitstellen einer alkalischen Silicatlösung mit einem pH ≥ 9,5 und das Senken des pH auf zwischen 3,0 und 5,0, bevor sie bei einem Individuum aufgebracht wird, umfasst, wobei die Anwendung der Antitranspirantzusammensetzung bei dem Individuum eine pH-Veränderung der Zusammensetzung bewirkt, die das Wachstum der Polykieselsäuren zur Bildung eines Gels induziert, wodurch ein Antitranspirant-Wirkstoff bereitgestellt wird, wobei die Polykieselsäure in Form von Polykieselsäure-Nanopartikeln mit mittleren Durchmessern von 20 nm oder weniger vorhanden ist und wobei die Vorrichtung aus einer Aerosolvorrichtung, einer Pumpspenderflasche, einem Roll-on, einer Vorrichtung mit einer perforierten Wand oder einem Stäbchen (Stick) ausgewählt ist;
wobei der pH über einen Zeitraum von unter 60 s gesenkt wird; und/oder
wobei die Polykieselsäurezusammensetzung durch einen Wachstumshemmer stabilisiert wird, wobei der Wachstumshemmer eine Carbonsäure, eine Aminosäure, ein anorganisches Anion, ein Polyol, Saccharid oder ein quartäres Ammoniumkation ist; und/oder
wobei die Polykieselsäurezusammensetzung durch ein mehrwertiges Kation und/oder ein nichtwässriges Lösungsmittel stabilisiert wird.

2. Nichttherapeutisches kosmetisches Verfahren zur Behandlung von menschlichem Körpergeruch unter Verwendung einer Antitranspirantzusammensetzung, umfassend eine stabilisierte Silicatzusammensetzung, die Polykieselsäuren umfasst, wobei die Antitranspirantzusammensetzung durch ein Verfahren erhältlich ist, das das Bereitstellen einer alkalischen Silicatlösung mit einem pH ≥ 9,5 und das Senken des pH auf zwischen 3,0 und 5,0, bevor sie bei einem Individuum aufgebracht wird, umfasst, wobei die Anwendung der Antitranspirantzusammensetzung bei dem Individuum eine pH-Veränderung der Zusammensetzung bewirkt, die das Wachstum der Polykieselsäuren zur Bildung eines Gels induziert, wodurch ein Antitranspirant-Wirkstoff bereitgestellt wird, wobei die Polykieselsäure in Form von Polykieselsäure-Nanopartikeln mit mittleren Durchmessern von 20 nm oder weniger vorhanden ist;
wobei der pH über einen Zeitraum von unter 60 s gesenkt wird; und/oder
wobei die Polykieselsäurezusammensetzung durch einen Wachstumshemmer stabilisiert wird, wobei der Wachstumshemmer eine Carbonsäure, eine Aminosäure, ein anorganisches Anion, ein Polyol, Saccharid oder ein quartäres Ammoniumkation ist; und/oder
wobei die Polykieselsäurezusammensetzung durch ein mehrwertiges Kation und/oder ein nichtwässriges Lösungsmittel stabilisiert wird.

3. Antitranspirantzusammensetzung zur Verwendung in einem Verfahren zur Behandlung eines medizinischen Leidens, das durch exzessive Transpiration gekennzeichnet ist, unter Verwendung einer Antitranspirantzusammensetzung, umfassend eine stabilisierte Silicatzusammensetzung, die Polykieselsäuren umfasst, wobei die Antitranspirantzusammensetzung durch ein Verfahren erhältlich ist, das das Bereitstellen einer alkalischen Silicatlösung mit einem pH ≥ 9,5 und das Senken des pH auf zwischen 3,0 und 5,0, bevor sie bei einem Individuum aufgebracht wird, umfasst, wobei die Anwendung der Antitranspirantzusammensetzung bei dem Individuum eine pH-Veränderung der Zusammensetzung bewirkt, die das Wachstum der Polykieselsäuren zur Bildung eines Gels induziert, wodurch ein Antitranspirant-Wirkstoff bereitgestellt wird, wobei die Polykieselsäure in Form von Polykieselsäure-Nanopartikeln mit mittleren Durchmessern von 20 nm oder weniger vorhanden ist;
wobei der pH über einen Zeitraum von unter 60 s gesenkt wird; und/oder
wobei die Polykieselsäurezusammensetzung durch einen Wachstumshemmer stabilisiert wird, wobei der Wachstumshemmer eine Carbonsäure, eine Aminosäure, ein anorganisches Anion, ein Polyol, Saccharid oder ein quartäres Ammoniumkation ist; und/oder
wobei die Polykieselsäurezusammensetzung durch ein mehrwertiges Kation und/oder ein nicht wässriges Lösungsmittel stabilisiert wird.

4. Vorrichtung, nichttherapeutisches kosmetisches Verfahren oder Zusammensetzung zur Verwendung in einem Verfahren zur Behandlung nach einem der Ansprüche 1 bis 3, wobei die Polykieselsäurezusammensetzung durch einen Wachstumshemmer stabilisiert wird, wobei der Wachstumshemmer eine Carbonsäure, eine Aminosäure, ein anorganisches Anion, ein Polyol, Saccharid oder ein quartäres Ammoniumkation ist.

5. Vorrichtung, nichttherapeutisches kosmetisches Verfahren oder Zusammensetzung zur Verwendung in einem Verfahren zur Behandlung nach einem der Ansprüche 1 bis 4, wobei die pH-Veränderung zu einem pH über 5 und unter 8, bevorzugt 5,5 bis 6,5 und noch bevorzugter zu einem pH von etwa 6,0, erfolgt.

6. Vorrichtung, nichttherapeutisches kosmetisches Verfahren oder Zusammensetzung zur Verwendung in einem Verfahren zur Behandlung nach einem der Ansprüche 1 bis 5, wobei die pH-Veränderung in den Hautporen erfolgt und die Produktion des Silicatgels in den Poren bewirkt.

7. Vorrichtung, nichttherapeutisches kosmetisches Verfahren oder Zusammensetzung zur Verwendung in einem Verfahren zur Behandlung nach einem der Ansprüche 1 bis 6, wobei der pH der Zusammensetzung vor der Anwendung bei dem Individuum gegebenenfalls zwischen pH 3,0 bis 4,5 oder gegebenenfalls zwischen 3,0 und 4,5 oder gegebenenfalls zwischen pH 3,5 und 4,0 liegt.

8. Vorrichtung, nichttherapeutisches kosmetisches Verfahren oder Zusammensetzung zur Verwendung in einem Verfahren zur Behandlung nach einem der Ansprüche 1 bis 7, wobei das stabilisierte Silicat durch zumindest einen Wachstumshemmer stabilisiert wird und gegebenenfalls durch zwei, drei, vier oder fünf Wachstumshemmer stabilisiert wird, wobei der/die Wachstumshemmer aus einer Carbonsäure, einer Aminosäure, einem anorganischen Anion, einem Polyol, Saccharid oder einem quartären Ammoniumkation ausgewählt ist/sind.

9. Vorrichtung, nichttherapeutisches kosmetisches Verfahren oder Zusammensetzung zur Verwendung in einem Verfahren zur Behandlung nach einem der Ansprüche 1 bis 8, wobei die Carbonsäure eine C₂₋₁₀-Carbonsäure ist und bevorzugt eine Dicarbonsäure wie Oxalsäure, Malonsäure, Gluconsäure, Glutarsäure, Weinsäure, Bernsteinsäure, Adipinsäure oder Pimelinsäure ist, die Aminosäure Asparaginsäure ist, ein Polyol etwa ein monomeres Polyol wie etwa Glycerin, Ethylenglykol, Xylit, Propylenglykol oder ein Polyalkylenglykol wie etwa Polyethylenglykol oder Polypropylenglykol ist oder das Polyol ein Saccharid ist, wie etwa Glucose, Fructose, Mannose, Saccharose, Threit, Erythrit, Sorbit, Dextran, Mannit, Dextran, Galactit oder Adonit, oder das quartäre Ammoniumkation Cholin ist.

10. Vorrichtung, nichttherapeutisches kosmetisches Verfahren oder Zusammensetzung zur Verwendung in einem Verfahren zur Behandlung nach einem der Ansprüche 1 bis 9, wobei:
(a) die Antitranspirantzusammensetzung ein oder mehrere Polyalkylenglykole umfasst; oder
(b) die Antitranspiratzusammensetzung Polyethylenglykol (PEG) umfasst.

11. Vorrichtung, nichttherapeutisches kosmetisches Verfahren oder Zusammensetzung zur Verwendung in einem Verfahren zur Behandlung nach einem der Ansprüche 1 bis 10, wobei die Polykieselsäurepartikel kaum kondensiert sind, wie in einem In-vitro-Auflösungstest bestimmt wurde, bei dem sich zumindest 25 % oder zumindest 30 % oder zumindest 35 % oder zumindest 40 % oder zumindest 50 % der Zusammensetzung innerhalb von 24 h in HEPES-Puffer auflöst.

12. Vorrichtung, nichttherapeutisches kosmetisches Verfahren oder Zusammensetzung zur Verwendung in einem Verfahren zur Behandlung nach Anspruch 10, wobei der In-vitro-Auflösungstest ein Molybdänsäuretest zur Bestimmung des löslichen Kieselsäureanteils ist.

13. Vorrichtung, nichttherapeutisches kosmetisches Verfahren oder Zusammensetzung zur Verwendung in einem Verfahren zur Behandlung nach einem der Ansprüche 1 bis 12, wobei die stabilisierte Silicatzusammensetzung ein mehrwertiges Kation wie etwa Ca²⁺, Mg²⁺, Cu²⁺, Fe³⁺ und/oder Zn²⁺ umfasst, wobei das mehrwertige Kation gegebenenfalls zugegeben wird, um eine Endkonzentration zwischen 0,01 M und 1,0 M und vorzugsweise eine Endkonzentration zwischen 0,05 M und 0,5 M bereitzustellen.

14. Vorrichtung, nichttherapeutisches kosmetisches Verfahren oder Zusammensetzung zur Verwendung in einem Verfahren zur Behandlung nach einem der Ansprüche 1 bis 13, wobei die stabilisierte Silicatzusammensetzung durch ein nichtwässriges Lösungsmittel wie etwa einen Alkohol stabilisiert wird, wobei der Alkohol gegebenenfalls Ethanol ist und/oder zwischen 10 und 70 Vol.-% des nichtwässrigen Lösungsmittels zugegeben wird.

15. Vorrichtung, nichttherapeutisches kosmetisches Verfahren oder Zusammensetzung zur Verwendung in einem Verfahren zur Behandlung nach einem der Ansprüche 1 bis 14, wobei die Zusammensetzung eines oder mehrere aus einem zusätzlichen Antitranspirant-Wirkstoff, einem Deodorant, einem flüchtigen oder nichtflüchtigen Öl, weichmachenden Ölen auf Silikon- und Kohlenwasserstoffbasis, einem Suspensionsmittel, einem Parfum oder Duftstoff, einem organischen Pulver und/oder einer mit Wasser nicht mischbaren organischen flüssigen Phase und zumindest einem Mittel zur Strukturierung dieser Phase umfasst.

16. Vorrichtung, nichttherapeutisches kosmetisches Verfahren oder Zusammensetzung zur Verwendung in einem Verfahren zur Behandlung nach einem der Ansprüche 1 bis 15, wobei die Zusammensetzung keine Aluminium- oder Zirkoniumsalze umfasst.

17. Antitranspirantzusammensetzung zur Verwendung in einem Verfahren zur Behandlung nach einem der Ansprüche 3 bis 16, wobei das medizinische Leiden Hyperhidrose ist, wobei die Hyperhidrose gegebenenfalls primäre oder fokale Hyperhidrose oder sekundäre Hyperhidrose ist.

18. Antitranspirantzusammensetzung zur Verwendung in einem Verfahren zur Behandlung nach einem der Ansprüche 3 bis 17, wobei die exzessive Transpiration durch Akromegalie, Angststörungen, Krebs, einschließlich Leukämie und Non-Hodgkin-Lymphom, Karzinoidsyndrom, Endokarditis, Herzinfarkt, Hyperthyreose, Drogenmissbrauch, Fettleibigkeit, Diabetes, Herzerkrankung, HIV/AIDS, Hyperthyreose, Lungenerkrankung, Medikamente wie Betablocker und trizyklische Antidepressiva, Menopause, Morbus Parkinson, Phäochromozytom, Rückenmarksverletzungen, Schlaganfall, Stress, Tuberkulose, Fieber oder eine Infektion verursacht wird.

19. Verfahren zur Herstellung einer Antitranspirantzusammensetzung, umfassend stabilisierte Polykieselsäuren, wobei das Verfahren Folgendes umfasst:
(a) Herstellen einer alkalischen Silicatlösung mit einem pH ≥ 9,5;
(b) gegebenenfalls Zugeben eines Wachstumshemmers zu der alkalischen Silicatlösung;
(c) Senken des pH auf ≤ 4,0 durch die Zugabe einer Säure, um eine Zusammensetzung zu bilden, die Polykieselsäuren umfasst;
(d) gegebenenfalls Zugeben eines mehrwertigen Kations und/oder eines Wachstumshemmers;
(e) Erhöhen des pH der Zusammensetzung auf einen physiologisch annehmbaren pH durch die Zugabe einer Base, wodurch die stabilisierte Zusammensetzung, die Polykieselsäuren umfasst, gebildet wird;
(f) gegebenenfalls Zugeben eines Wachstumshemmers, der in der Lage ist, die Stabilität der Zusammensetzung zu erhöhen, wobei der Wachstumshemmer eine Carbonsäure, eine Aminosäure, ein anorganisches Anion, ein Polyol, Saccharid oder ein quartäres Ammoniumkation ist; und
(g) gegebenenfalls Zugeben eines nichtwässrigen Lösungsmittels, das in der Lage ist, die Stabilität der Zusammensetzung zu erhöhen; und
(h) Integrieren der Polykieselsäurezusammensetzung in eine Antitranspirantzusammensetzung;
wobei die Polykieselsäure in Form von Polykieselsäure-Nanopartikeln mit mittleren Durchmessern von 20 nm oder weniger vorhanden ist und die Antitranspirantzusammensetzung einen pH zwischen 3,0 und 5,0 aufweist.

20. Verfahren nach Anspruch 19, wobei
(i) der pH in Schritt (c) über einen Zeitraum von weniger als 60 s, weniger als 30 s, weniger als 10 s oder weniger als 5 s gesenkt wird; und/oder
(ii) der pH in Schritt (c) auf einen pH ≤ 3,0 gesenkt wird; und/oder
(iii) die Konzentration der Silikatlösung zwischen 0,05 M und 3,0 M liegt, wobei die Konzentration der Silikatlösung gegebenenfalls zwischen 0,1 M und 1,5 M liegt; und/oder
(iv) die Silikatzusammensetzung 6 Monate oder länger, 12 Monate oder länger oder 24 Monate oder länger stabil ist; und/oder
(v) die Polykieselsäurepartikel einen mittleren Durchmesser von unter 20 nm, gegebenenfalls 10 nm oder weniger, gegebenenfalls 5 nm oder weniger und gegebenenfalls zwischen 1 und 3 nm, aufweisen; und/oder
(vi) die stabilisierte Silikatzusammensetzung eine bei 25 °C gemessene Viskosität von weniger als 4,0 mPa·s aufweist; und/oder
(vii) der pH der alkalischen Silikatlösung in Schritt (a) etwa 11,5 beträgt; und/oder
(viii) zumindest ein Wachstumshemmer in Schritt (b) zugegeben wird und wobei gegebenenfalls zwei, drei, vier oder fünf Wachstumshemmer in Schritt (b) zugegeben werden; und/oder
(ix) der Wachstumshemmer eine Carbonsäure, eine Aminosäure, ein anorganisches Anion, ein Polyol (wie etwa ein Polyalkylenglykol), Saccharid und/oder ein quartäres Ammoniumkation ist und wobei gegebenenfalls die Carbonsäure eine C₂₋₁₀-Carbonsäure ist und bevorzugt eine Dicarbonsäure wie Oxalsäure, Malonsäure, Gluconsäure, Glutarsäure, Weinsäure, Bernsteinsäure, Adipinsäure oder Pimelinsäure ist, die Aminosäure Asparaginsäure ist, ein Polyol wie etwa ein monomeres Polyol wie etwa Glycerin, Ethylenglykol, Xylit, Propylenglykol oder ein Polyalkylenglykol wie etwa Polyethylenglykol oder Polypropylenglykol ist oder das Polyol ein Saccharid ist, wie etwa Glucose, Fructose, Mannose, Saccharose, Threit, Erythrit, Sorbit, Dextran, Mannit, Dextran, Galactit oder Adonit, oder das quartäre Ammoniumkation Cholin ist; und/oder
(x) der pH der Zusammensetzung in Schritt (c) auf einen pH ≤ 1,5 gesenkt wird; und/oder
(xi) das mehrwertige Kation Ca²⁺, Mg²⁺, Cu²⁺, Fe³⁺ und/oder Zn²⁺ ist; und/oder
(xii) das mehrwertige Kation zugegeben wird, um eine Endkonzentration zwischen 0,01 M und 1,0 M bereitzustellen, und wobei das mehrwertige Kation gegebenenfalls zugegeben wird, um eine Endkonzentration zwischen 0,05 M und 0,5 M bereitzustellen; und/oder
(xiii) der pH der Zusammensetzung in Schritt (e) auf einen pH zwischen 3,0 und 5,0 erhöht wird und wobei der pH der Zusammensetzung in Schritt (e) gegebenenfalls auf einen pH zwischen 3,5 und 4,0 erhöht wird, wobei der pH gegebenenfalls unter Verwendung einer Base erhöht wird, die Natriumhydroxid oder Natriumcarbonat ist; und/oder
(xiv) das nichtwässrige Lösungsmittel ein Alkohol ist, wobei der Alkohol gegebenenfalls Ethanol ist und/oder zwischen 10 und 70 Vol.-% des nichtwässrigen Lösungsmittels zugegeben wird.

21. Verfahren nach Anspruch 19 oder Anspruch 20, wobei das Verfahren den weiteren Schritt des Formulierens der stabilisierten Silikatzusammensetzung mit einem oder mehreren zusätzlichen Bestandteilen umfasst, um eine Antitranspirantzusammensetzung herzustellen.

22. Verfahren nach Anspruch 21, wobei die stabilisierten Polykieselsäurepartikel in eine Antitranspirantzusammensetzung oder eine kosmetische Formulierung, umfassend ein oder mehrere Polyalkylenglykole, integriert werden und wobei die stabilisierten Polykieselsäurepartikel gegebenenfalls in eine Antitranspirantzusammensetzung oder eine kosmetische Formulierung, umfassend Polyethylenglykol (PEG), integriert werden.

23. Verfahren nach Anspruch 21 oder Anspruch 22, wobei die zusätzlichen Bestandteile eines oder mehrere aus einem zusätzlichen Antitranspirant-Wirkstoff, einem Deodorant, einem flüchtigen oder nichtflüchtigen Öl, weichmachenden Ölen auf Silikon- und Kohlenwasserstoffbasis, einem Suspensionsmittel, einem Parfum oder Duftstoff, einem organischen Pulver und/oder einer mit Wasser nicht mischbaren organischen flüssigen Phase und zumindest einem Mittel zur Strukturierung dieser Phase umfassen.

## Revendications

1. Dispositif pour distribuer une composition antitranspirante comprenant une composition de silicate stabilisée qui comprend des acides polysiliciques, dans lequel la composition antitranspirante peut être obtenue par un procédé comprenant la fourniture d'une solution de silicate alcaline ayant un pH ≥ 9,5 et la diminution du pH entre 3,0 et 5,0 avant application à un sujet, dans lequel l'application de la composition antitranspirante au sujet cause un changement de pH dans la composition qui induit la croissance des acides polysiliciques pour former un gel de façon à fournir une substance active antitranspirante, dans lequel l'acide polysilicique est présent sous la forme de nanoparticules d'acide polysilicique ayant des diamètres moyens de 20 nm ou moins, et le dispositif étant choisi parmi un dispositif d'aérosol, un flacon à bouchon gicleur, une bille roulante, un dispositif équipé d'une paroi perforée, ou un bâtonnet (stick) ;
dans lequel le pH est diminué pendant une durée inférieure à 60 secondes ; et/ou
dans lequel la composition d'acide polysilicique est stabilisée par un retardateur de croissance, dans lequel le retardateur de croissance est un acide carboxylique, un acide aminé, un anion inorganique, un polyol, un saccharide ou un cation ammonium quaternaire ; et/ou
dans lequel la composition d'acide polysilicique est stabilisée par un cation multivalent et/ou par un solvant non aqueux.

2. Procédé cosmétique non thérapeutique pour traiter une odeur d'un corps humain au moyen d'une composition antitranspirante comprenant une composition de silicate stabilisée qui comprend des acides polysiliciques, dans lequel la composition antitranspirante peut être obtenue par un procédé comprenant la fourniture d'une solution de silicate alcaline ayant un pH ≥ 9,5 et la diminution du pH entre 3,0 et 5,0 avant application à un sujet, dans lequel l'application de la composition antitranspirante au sujet cause un changement de pH dans la composition qui induit la croissance des acides polysiliciques pour former un gel de façon à fournir une substance active antitranspirante, dans lequel l'acide polysilicique est présent sous la forme de nanoparticules d'acide polysilicique ayant des diamètres moyens de 20 nm ou moins ;
dans lequel le pH est diminué pendant une durée inférieure à 60 secondes ; et/ou
dans lequel la composition d'acide polysilicique est stabilisée par un retardateur de croissance, dans lequel le retardateur de croissance est un acide carboxylique, un acide aminé, un anion inorganique, un polyol, un saccharide ou un cation ammonium quaternaire ; et/ou
dans lequel la composition d'acide polysilicique est stabilisée par un cation multivalent et/ou par un solvant non aqueux.

3. Composition antitranspirante pour utilisation dans un procédé de traitement d'une affection médicale **caractérisé par** une transpiration excessive au moyen d'une composition antitranspirante comprenant une composition de silicate stabilisée qui comprend des acides polysiliciques, la composition antitranspirante pouvant être obtenue par un procédé comprenant la fourniture d'une solution de silicate alcaline ayant un pH ≥ 9,5 et la diminution du pH entre 3,0 et 5,0 avant application à un sujet, l'application de la composition antitranspirante au sujet causant un changement de pH dans la composition qui induit la croissance des acides polysiliciques pour former un gel de façon à fournir une substance active antitranspirante, dans laquelle l'acide polysilicique est présent sous la forme de nanoparticules d'acide polysilicique ayant des diamètres moyens de 20 nm ou moins ;
dans laquelle le pH est diminué pendant une durée inférieure à 60 secondes ; et/ou
dans laquelle la composition d'acide polysilicique est stabilisée par un retardateur de croissance, dans laquelle le retardateur de croissance est un acide carboxylique, un acide aminé, un anion inorganique, un polyol, un saccharide ou un cation ammonium quaternaire ; et/ou
dans laquelle la composition d'acide polysilicique est stabilisée par un cation multivalent et/ou par un solvant non aqueux.

4. Dispositif, procédé cosmétique non thérapeutique ou composition pour utilisation dans un procédé de traitement selon l'une quelconque des revendications 1 à 3, dans lesquels la composition d'acide polysilicique est stabilisée par un retardateur de croissance, dans lesquels le retardateur de croissance est un acide carboxylique, un acide aminé, un anion inorganique, un polyol, un saccharide ou un cation ammonium quaternaire.

5. Dispositif, procédé cosmétique non thérapeutique ou composition pour utilisation dans un procédé de traitement selon l'une quelconque des revendications 1 à 4, dans lesquels le changement de pH est à un pH supérieur à 5 et inférieur à 8, de préférence 5,5 à 6,5, et plus préférablement à un pH d'environ 6,0.

6. Dispositif, procédé cosmétique non thérapeutique ou composition pour utilisation dans un procédé de traitement selon l'une quelconque des revendications 1 à 5, dans lesquels le changement de pH se produit dans les pores de la peau et cause la production du gel de silicate dans les pores.

7. Dispositif, procédé cosmétique non thérapeutique ou composition pour utilisation dans un procédé de traitement selon l'une quelconque des revendications 1 à 6, dans lesquels le pH de la composition avant application au sujet est facultativement de pH 3,0 à 4,5, ou facultativement entre pH 3,0 et 4,5, ou facultativement entre pH 3,5 et 4,0.

8. Dispositif, procédé cosmétique non thérapeutique ou composition pour utilisation dans un procédé de traitement selon l'une quelconque des revendications 1 à 7, dans lesquels le silicate stabilisé est stabilisé par au moins un retardateur de croissance, et facultativement par deux, trois, quatre ou cinq retardateurs de croissance, le(s) retardateur(s) de croissance est/sont choisi(s) parmi un acide carboxylique, un acide aminé, un anion inorganique, un polyol, un saccharide et un cation ammonium quaternaire.

9. Dispositif, procédé cosmétique non thérapeutique ou composition pour utilisation dans un procédé de traitement selon l'une quelconque des revendications 1 à 8, dans lesquels l'acide carboxylique est un acide carboxylique en C₂₋₁₀, et de préférence un acide dicarboxylique tel que l'acide oxalique, l'acide malonique, l'acide gluconique, l'acide glutarique, l'acide tartrique, l'acide succinique, l'acide adipique acide ou l'acide pimélique, l'acide aminé est l'acide aspartique, un polyol tel qu'un polyol monomère, tel que le glycérol, l'éthylène glycol, le xylitol, le propylène glycol, ou un polyalkylène glycol, tel que le polyéthylène glycol ou le polypropylène glycol, ou le polyol est un saccharide, tel que le glucose, le fructose, le mannose, le saccharose, le thréitol, l'érythritol, le sorbitol, le dextrane, le mannitol, le dextrane, le galactitol ou l'adonitol, ou le cation ammonium quaternaire est la choline.

10. Dispositif, procédé cosmétique non thérapeutique ou composition pour utilisation dans un procédé de traitement selon l'une quelconque des revendications 1 à 9, dans lesquels :
(a) la composition antitranspirante comprend un ou plusieurs polyalkylène glycols ; ou
(b) ladite composition antitranspirante comprend du polyéthylène glycol (PEG).

11. Dispositif, procédé cosmétique non thérapeutique ou composition pour utilisation dans un procédé de traitement selon l'une quelconque des revendications 1 à 10, dans lesquels les particules d'acide polysilicique sont faiblement condensées comme déterminé dans un essai de dissolution *in vitro* dans lequel au moins 25 %, ou au moins 30 %, ou au moins 35 %, ou au moins 40 %, ou au moins 50 % de la composition est dissoute en 24 heures dans du tampon HEPES.

12. Dispositif, procédé cosmétique non thérapeutique ou composition pour utilisation dans un procédé de traitement selon la revendication 10, dans lesquels l'essai de dissolution *in vitro* est un essai à l'acide molybdique pour déterminer la fraction soluble d'acide silicique.

13. Dispositif, procédé cosmétique non thérapeutique ou composition pour utilisation dans un procédé de traitement selon l'une quelconque des revendications 1 à 12, dans lesquels la composition de silicate stabilisée comprend un cation multivalent tel que Ca²⁺, Mg²⁺, Cu²⁺, Fe³⁺ et/ou Zn²⁺, facultativement dans lesquels le cation multivalent est ajouté pour obtenir une concentration finale comprise entre 0,01 M et 1,0 M, et de préférence une concentration finale comprise entre 0,05 M et 0,5 M.

14. Dispositif, procédé cosmétique non thérapeutique ou composition pour utilisation dans un procédé de traitement selon l'une quelconque des revendications 1 à 13, dans lesquels la composition de silicate stabilisée est stabilisée par un solvant non aqueux, tel qu'un alcool, facultativement dans lesquels l'alcool est l'éthanol et/ou le solvant non aqueux est ajouté entre 10 et 70 % v/v.

15. Dispositif, procédé cosmétique non thérapeutique ou composition pour utilisation dans un procédé de traitement selon l'une quelconque des revendications 1 à 14, dans lesquels la composition comprend l'un ou plusieurs parmi un agent actif antitranspirant supplémentaire, un déodorant, une huile volatile ou non volatile, des huiles émollientes à base de silicone et d'hydrocarbures, un agent de suspension, un parfum ou une fragrance, une poudre organique et/ou une phase liquide organique non miscible dans l'eau et au moins un agent pour structurer ladite phase.

16. Dispositif, procédé cosmétique non thérapeutique ou composition pour utilisation dans un procédé de traitement selon l'une quelconque des revendications 1 à 15, dans lesquels la composition ne comprend pas de sels d'aluminium ou de zirconium.

17. Composition antitranspirante pour utilisation dans un procédé de traitement des revendications 3 à 16, l'affection médicale étant l'hyperhidrose, l'hyperhidrose étant facultativement une hyperhidrose primitive ou focale ou une hyperhidrose secondaire.

18. Composition antitranspirante pour utilisation dans un procédé de traitement des revendications 3 à 17, la transpiration excessive étant causée par une acromégalie, des troubles anxieux, un cancer, comprenant une leucémie et un lymphome non hodgkinien, un syndrome carcinoïde, l'endocardite, une attaque cardiaque, l'hyperthyroïdie, une toxicomanie, l'obésité, le diabète, une maladie cardiaque, le VIH/SIDA, l'hyperthyroïdie, une maladie pulmonaire, des médicaments, tels que des bêta-bloquants et des antidépresseurs tricycliques, la ménopause, la maladie de Parkinson, le phéochromocytome, une lésion de la moelle épinière, un accident vasculaire cérébral, le stress, la tuberculose, la fièvre ou une infection.

19. Procédé de production d'une composition antitranspirante qui comprend des acides polysiliciques stabilisés, le procédé comprenant :
(a) la préparation d'une solution de silicate alcaline ayant un pH ≥ 9,5 ;
(b) facultativement l'ajout d'un retardateur de croissance à la solution de silicate alcaline ;
(c) la diminution du pH à ≤ 4,0 par ajout d'un acide pour former une composition comprenant des acides polysiliciques ;
(d) facultativement l'ajout d'un cation multivalent et/ou d'un retardateur de croissance ;
(e) l'augmentation du pH de la composition à un pH physiologique acceptable par ajout d'une base, de façon à former la composition stabilisée comprenant des acides polysiliciques ;
(f) facultativement l'ajout d'un retardateur de croissance capable d'augmenter la stabilité de la composition, dans lequel le retardateur de croissance est un acide carboxylique, un acide aminé, un anion inorganique, un polyol, un saccharide ou un cation ammonium quaternaire ; et,
(g) facultativement l'ajout d'un solvant non aqueux capable d'augmenter la stabilité de la composition ; et
(h) l'incorporation de la composition d'acide polysilicique dans une formulation antitranspirante ;
dans lequel l'acide polysilicique est présent sous la forme de nanoparticules d'acide polysilicique ayant des diamètres moyens de 20 nm ou moins et la composition antitranspirante a un pH compris entre 3,0 et 5,0.

20. Procédé selon la revendication 19, dans lequel :
(i) dans l'étape (c) le pH est diminué pendant une durée inférieure à 60 secondes, inférieure à 30 secondes, inférieure à 10 secondes, ou inférieure à 5 secondes ; et/ou
(ii) dans l'étape (c), le pH est diminué à un pH ≤ 3,0 ; et/ou
(iii) la concentration de la solution de silicate est comprise entre 0,05 M et 3,0 M, facultativement dans lequel la concentration de la solution de silicate est comprise entre 0,1 M et 1,5 M ; et/ou
(iv) la composition de silicate est stable pendant 6 mois ou plus, 12 mois ou plus ou 24 mois ou plus ; et/ou
(v) les particules d'acide polysilicique ont un diamètre moyen inférieur à 20 nm, facultativement de 10 nm ou moins, facultativement de 5 nm ou moins et facultativement compris entre 1 et 3 nm ; et/ou
(vi) la composition de silicate stabilisée a une viscosité mesurée à 25 °C inférieure à 4,0 mPa·s ; et/ou
(vii) dans l'étape (a), le pH de la solution de silicate alcaline est d'environ 11,5 ; et/ou
(viii) au moins un retardateur de croissance est ajouté dans l'étape (b), et facultativement dans lequel deux, trois, quatre ou cinq retardateurs de croissance sont ajoutés dans l'étape (b) ; et/ou
(ix) le retardateur de croissance est un acide carboxylique, un acide aminé, un anion inorganique, un polyol (tel qu'un polyalkylène glycol), un saccharide et/ou un cation ammonium quaternaire, et facultativement dans lequel l'acide carboxylique est un acide carboxylique en C₂₋₁₀, et de préférence un acide dicarboxylique tel que l'acide oxalique, l'acide malonique, l'acide gluconique, l'acide glutarique, l'acide tartrique, l'acide succinique, l'acide adipique acide ou l'acide pimélique, l'acide aminé est l'acide aspartique, un polyol tel qu'un polyol monomère, tel que le glycérol, l'éthylène glycol, le xylitol, le propylène glycol, ou un polyalkylène glycol, tel que le polyéthylène glycol ou le polypropylène glycol, ou le polyol est un saccharide, tel que le glucose, le fructose, le mannose, le saccharose, le thréitol, l'érythritol, le sorbitol, le dextrane, le mannitol, le dextrane, le galactitol ou l'adonitol, ou le cation ammonium quaternaire est la choline ; et/ou
(x) dans l'étape (c), le pH de la composition est diminué à un pH ≤ 1,5 ; et/ou
(xi) le cation multivalent est Ca²⁺, Mg²⁺, Fe³⁺, Cu²⁺ et/ou Zn²⁺ ; et/ou
(xii) le cation multivalent est ajouté pour obtenir une concentration finale comprise entre 0,01 M et 1,0 M, et facultativement, dans lequel le cation multivalent est ajouté pour obtenir une concentration finale comprise entre 0,05 M et 0,5 M ; et/ou
(xiii) dans l'étape (e), le pH de la composition est augmenté à un pH compris entre 3,0 et 5,0, et facultativement dans lequel, dans l'étape (e), le pH de la composition est augmenté à un pH compris entre 3,5 et 4,0, facultativement dans lequel le pH est augmenté au moyen d'une base qui est l'hydroxyde de sodium ou le carbonate de sodium ; et/ou
(xiv) le solvant non aqueux est un alcool, et facultativement, dans lequel l'alcool est l'éthanol et/ou le solvant non aqueux est ajouté entre 10 et 70 % v/v.

21. Procédé selon la revendication 19 ou la revendication 20, le procédé comprenant l'étape supplémentaire de formulation de la composition de silicate stabilisée avec un ou plusieurs composants supplémentaires pour produire une composition antitranspirante.

22. Procédé selon la revendication 21, dans lequel les particules d'acide polysilicique stabilisé sont incorporées dans une composition antitranspirante ou une formulation cosmétique comprenant un ou plusieurs polyalkylène glycols, et facultativement dans lequel les particules d'acide polysilicique stabilisé sont incorporées dans une composition antitranspirante ou une formulation cosmétique comprenant du polyéthylène glycol (PEG).

23. Procédé selon la revendication 21 ou la revendication 22, dans lequel les composants supplémentaires comprennent l'un ou plusieurs parmi un agent actif antitranspirant supplémentaire, un déodorant, une huile choisie parmi des huiles volatiles ou non volatiles et des huiles émollientes à base de silicone et d'hydrocarbures, un agent de suspension, une poudre organique et/ou une phase liquide organique non miscible dans l'eau et au moins un agent pour structurer ladite phase.
